(19) Europäisches Patentamt
European Patent Office
Office européen des brevets

(11) EP 2 111 391 B1

(12) FASCICULE DE BREVET EUROPEEN

(45) Date de publication et mention de la délivrance du brevet:
**31.10.2012 Bulletin 2012/44**

(21) Numéro de dépôt: **08701535.0**

(22) Date de dépôt: **16.01.2008**

(51) Int Cl.:
*C07C 227/00* (2006.01) *C07C 229/30* (2006.01)
*C07C 237/16* (2006.01) *A61K 8/44* (2006.01)
*A61K 31/197* (2006.01) *A61Q 19/08* (2006.01)
*A61P 17/00* (2006.01) *A61Q 5/10* (2006.01)

(86) Numéro de dépôt international:
**PCT/EP2008/050471**

(87) Numéro de publication internationale:
**WO 2008/090073 (31.07.2008 Gazette 2008/31)**

# (54) NOUVEAUX DERIVES AMINO-ACIDES GRAS INSATURES ET LEUR UTILISATION DERMO COSMETOLOGIQUE

NEUE UNGESÄTTIGTE AMINOFETTSÄURE-DERIVATE UND IHRE VERWENDUNG IN DER HAUTKOSMETIK

NEW UNSATURATED FATTY AMINO-ACID DERIVATIVES AND USE THEREOF IN DERMAL COSMETOLOGY

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **16.01.2007 FR 0700291**
**15.03.2007 FR 0753864**

(43) Date de publication de la demande:
**28.10.2009 Bulletin 2009/44**

(73) Titulaire: **Pierre Fabre Dermo-Cosmétique**
**92100 Boulogne-Billancourt (FR)**

(72) Inventeurs:
- **TARROUX, Roger**
**F-31300 Toulouse (FR)**
- **CHARVERON, Marie**
**F-31000 Toulouse (FR)**
- **DAUNES-MARION, Sylvie**
**F-31000 Toulouse (FR)**
- **FRISON, Natacha**
**F-60100 Creil (FR)**
- **FOLLEAS, Benoît**
**F-60300 Senlis (FR)**
- **BRAYER, Jean-Louis**
**F-60440 Nanteuil Le Haudoin (FR)**

(74) Mandataire: **Ahner, Francis et al**
**Cabinet Regimbeau**
**20, rue de Chazelles**
**75847 Paris Cedex 17 (FR)**

(56) Documents cités:
**WO-A-91/04746**
**WO-A-03/022787**
**WO-A-2005/068643**

- **DAVIES ET AL: "Homochiral lithium amides for the asymmetric synthesis of beta-amino acids" TETRAHEDRON: ASYMMETRY, PERGAMON, OXFORD, GB, vol. 17, no. 12, 31 juillet 2006 (2006-07-31), pages 1793-1811, XP005586352 ISSN: 0957-4166**
- **G. MENTINK ET AL.: "Allenylmethylsilanes as Nucleophiles in N-Acyliminium Ion Chemistry" ORGANIC LETTERS, vol. 4, no. 20, 2002, pages 3497-3500, XP002455238 USAMERICAN CHEMICAL SOCIETY**
- **LIST B ET AL: "Practical Synthesis of (E)-alpha, beta-Unsaturated Esters from Aldehydes" ADVANCED SYNTHESIS AND CATALYSIS, WILEY-VCH, WEINHEIM, DE, vol. 347, 2005, pages 1558-1560, XP002390820 ISSN: 1615-4150**
- **C.H.STAMMER ET R.G. WEBB: "The synthesis of Racemic threo- and erythro-beta-Hydroxylisines" JOURNAL OF ORGANIC CHEMISTRY., vol. 34, no. 8, août 1969 (1969-08), pages 2306-2311, XP002455237 USAMERICAN CHEMICAL SOCIETY, WASHINGTON, DC.**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- MARSZAK I ET AL: "RECHERCHES SUR LES AMINOACIDES ET LEURS DERIVES. I. - SUR LA SYNTHESE DES AMINOACIDES A PARTIR DES AMINES TERTIAIRES A FONCTION ACETYLENIQUE VRAI AMINO ACIDS AND THEIR DERIVATIVES. I. THE SYNTHESIS OF AMINO ACIDS FROM TERTIARY AMNES HAVING A TRUE ACETYL" BULLETIN DE LA SOCIETE CHIMIQUE DE FRANCE, SOCIETE FRANCAISE DE CHIMIE. PARIS, FR, 1959, pages 182-185, XP009064176 ISSN: 0037-8968
- Y. GUINDON ET AL.: "Intramolecular Aminyl and Iminyl Radical Additions..." ORGANIC LETTERS, vol. 3, no. 15, 2001, pages 2293-2296, XP002455239 USAMERICAN CHEMICAL SOCIETY
- KNOUZI N ET AL: "INTRAMOLECULAR CYCLIZATION OF OMEGA-PRIMARY AMINO ELECTROPHILIC OLEFINS TO FUNCTIONALIZED PYRROLIDINES AND PIPERIDINES" TETRAHEDRON, ELSEVIER SCIENCE PUBLISHERS, AMSTERDAM, NL, vol. 28, no. 16, 1987, pages 1757-1760, XP002031799 ISSN: 0040-4020
- PERRON J ET AL: "First synthesis of pyrrolo[1,2: 1',2']azepino[5,6-b]indole derivatives" TETRAHEDRON LETTERS, ELSEVIER, AMSTERDAM, NL, vol. 44, no. 35, 25 août 2003 (2003-08-25), pages 6553-6556, XP004442799 ISSN: 0040-4039
- CACCIOLA J ET AL: "The Synthesis of Lysine alpha-Ketoamide Thrombin Inhibitors via an Epoxy AmideRing Opening" TETRAHEDRON LETTERS, ELSEVIER, AMSTERDAM, NL, vol. 38, no. 33, 18 août 1997 (1997-08-18), pages 5741-5744, XP004085859 ISSN: 0040-4039

## Description

La présente invention a pour objet de nouveaux amino-acides gras insaturés, ainsi que leur utilisation dermocosmétologique.

De tels amino-acides gras insaturés sont notamment décrits dans les documents suivants :

- WO 2005/068643 qui concerne la synthèse biochimique de l'acide 6-aminocaproïque utile comme intermédiaire de synthèse de l'acide 6-aminohex-2-énoïque, de l'acide 6-aminohexanoïque, du caprolactame et du nylon-6 ;
- l'article de Davies et al. (Tetrahedron : Asymmetry 2006, 17, 1793-1811) qui concerne la synthèse de β-amino-acides et qui décrit notamment le (*E*)-6-(*N*,*N*-di-tert-butoxy-carbonylamino)hex-2-énoate de tert-butyle comme intermédiaire de synthèse ;
- l'article de Mentink et al. (Org. Lett. 2002, 4(20), 3497-1500) qui concerne l'utilisation d'allénylméthylsilanes comme nucléophiles dans la chimie des ions N-acyliminium, ces nucléophiles ayant notamment été additionnés sur les amino-acides insaturés suivants: l'ester méthylique de l'acide 6-(2-éthoxy-5-oxo-pyrrolidin-1-yl)hex-2-énoïque et l'ester méthylique de l'acide 7-(2-éthoxy-5-oxo-pyrrolidin-1-yl)hept-2-énoïque ;
- l'article de Stammer et Webb (J. Org. Chem. 1969, 34(8), 2306-2311) qui concerne la synthèse de β-hydroxylysines utilisant notamment le chlorhydrate de l'acide 6-amino-trans-2-hexénoïque ;
- l'article de Marszak et Olomucki (Bull. Soc. Chim. Fr. 1959, 182-185) qui concerne la synthèse d'aminoacides à partir d'amines tertiaires à fonction acétylénique vrai, dont l'acide diméthylamino-8-octène-2-oïque et l'acide diméthylamino-12-dodécène-2-oïque ;
- l'article de Guindon et al. (Org. Lett. 2001, 3(15), 2293-2296) qui concerne l'addition intramoléculaire de radicaux aminyles et iminyles sur des esters α,β-insaturés, les esters éthyliques des acides 6-isopropylamino-2-méthyl-hex-2-énoïque et 6-*tert*-butylamino-2-méthyl-hex-2-énoïque étant notamment utilisés comme intermédiaires de synthèse des produits de départs utilisés pour réaliser cette réaction radicalaire ;
- l'article de Knouzi et al. (Tetrahedron Lett. 1987, 28(16), 1757-1760) qui concerne la synthèse de pyrrolidines et pipéridines fonctionnalisées, les esters méthyliques des acides 6-amino-2-méthyl-hex-2-énoïque, 7-amino-hept-2-énoïque, 7-amino-2-méthyl-hept-2-énoïque et 7-amino-4-méthyl-hept-2-énoïque ayant notamment été utilisés comme intermédiaires dans cette synthèse ;
- WO 91/04746 qui concerne des peptides et pseudopeptides anti-thrombotiques, l'acide 8-*N*-*tert*-butoxycarbonyl-amino-2-octénoïque et le trifluoroacétate de l'acide 8-amino-2-octénoïque ayant été utilisés comme intermédiaires de synthèse ;
- l'article de Perron et al. (Tetrahedron Lett. 2003, 44, 6553-6556) qui concerne la synthèse de dérivés de pyrrolo [1,2:1',2']azépino[5,6-b]indole, l'ester éthylique de l'acide 6-*N*-*tert*-butoxycarbonyl-amino-hex-2-énoïque ; et

l'article de Cacciola et al. (Tetrahedron Lett. 1997, 38(33), 5741-5744) qui concerne la synthèse d'inhibiteurs de thrombine de type α-céto-amide de lysine, l'acide 7-*N*-*tert*-butoxycarbonyl-amino-hept-2-énoïque et son ester méthylique étant décrits comme intermédiaires de synthèse.

Plus particulièrement, la présente invention a pour objet, à titre de médicaments, des dérivés d'amino-acides gras insaturés répondant à la formule générale (1) :

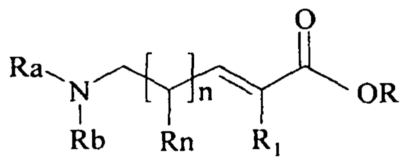

(I)

ainsi que leurs sels d'addition d'acides pharmaceutiquement acceptables, dans laquelle :

- Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;
- $R_1$ représente un atome d'hydrogène, de fluor, de chlore, ou de brome, ou bien un groupement $-CF_3$ ou $-CHF_2$ ou bien un groupement alkyle, alkényle ou alkynyle, linéaire ou ramifié, comprenant 1 à 6 atomes de carbone, et étant éventuellement substitué par un atome d'halogène, en particulier de fluor;
- R représente un atome d'hydrogène ou un groupement R' représentant un groupement alkyle, linéaire ou ramifié, comprenant 1 à 6 atomes de carbone, ou cycloalkyle comprenant 3 à 6 atomes de carbone, et étant éventuellement substitué par un atome d'halogène, en particulier de fluor ;

- Ra et Rb représentent indépendamment l'un de l'autre un atome d'hydrogène ou un groupement alkyle ou acyle, linéaire ou ramifié, comprenant 1 à 6 atomes de carbone, Ra et Rb pouvant former ensemble un cycle hydrocarboné comportant de 4 à 6 atomes de carbone ; et
- n est un entier compris entre 2 et 14.

Par groupement « alkyle », on entend une chaîne hydrocarbonée saturée, linéaire ou ramifiée, comportant de préférence de 1 à 6 atomes de carbone, comme par exemple un groupement, méthyle, éthyle, isopropyle, *tertio*-butyle, pentyle, etc.

Par groupement « alkényle », on entend une chaîne hydrocarbonée, linéaire ou ramifiée, comportant au moins une double liaison et comportant de préférence de 2 à 6 atomes de carbone, comme par exemple un groupement éthényle, propényle, 2,4-hexadiényle, etc.

Par groupement « alkynyle », on entend une chaîne hydrocarbonée, linéaire ou ramifiée, comportant au moins une triple liaison et comportant de préférence de 2 à 6 atomes de carbone, comme par exemple un groupement éthynyle, propynyle, 2,4-hexadiynyle, etc.

Par groupement « cycloalkyle », on entend un groupement hydrocarboné cyclique saturé, comportant de préférence de 3 à 6 atomes de carbone, comme par exemple un groupement cyclopropyle, cyclohexyle, cyclopentyle, etc.

Par groupement « acyle », on entend un groupement alkyl-carbonyle, c'est-à-dire un groupe alkyle tel que défini ci-dessus lié par l'intermédiaire d'un groupe carbonyle, comme par exemple un acétyle.

Par « halogène », on entend un fluor, un brome, un iode ou un chlore.

Selon une caractéristique particulière de l'invention, les dérivés d'amino-acides gras insaturés de formule générale (I) répondent aux critères suivants :

- Rn représentent un atome d'hydrogène sur tous les carbones de la chaîne carbonée sauf sur un où Rn représente un groupement alkyle, linéaire ou ramifié, comprenant 1 à 6 atomes de carbone, et étant éventuellement substitué par un ou plusieurs atomes d'halogène, en particulier de fluor ;
- R représente un atome d'hydrogène ou un groupement alkyle, linéaire ou ramifié, comprenant 1 à 6 atomes de carbone, ou cycloalkyle comprenant 3 à 6 atomes de carbone, et étant éventuellement substitué par un atome d'halogène, en particulier de fluor ; R représente de préférence un atome d'hydrogène ;
- n, Ra, Rb et $R_1$ sont tels que définis précédemment.

Selon une caractéristique particulière de l'invention, les dérivés d'amino-acides gras insaturés de formule générale (I) répondent aux critères suivants :

- Ra et Rb représentent un atome d'hydrogène.

Selon une caractéristique particulière de l'invention, les dérivés d'amino-acides gras insaturés de formule générale (1) répondent aux critères suivants :

- R représentent un atome d'hydrogène.

Selon une caractéristique particulière de l'invention, les dérivés d'amino-acides gras insaturés de formule générale (I) répondent aux critères suivants :

- Rn représentent un atome d'hydrogène.

Selon une caractéristique particulière de l'invention, les dérivés d'amino-acides gras insaturés de formule générale (I) répondent aux critères suivants :

- $R_1$ représente un atome d'hydrogène ou de fluor.

Selon une caractéristique particulière de l'invention, les dérivés d'amino-acides gras insaturés de formule générale (I) répondent aux critères suivants :

- n est compris entre 3 et 5.

Selon une caractéristique particulière de l'invention, les dérivés d'amino-acides gras insaturés de formule générale (I) répondent aux critères suivants :

• n est compris entre 9 et 14.

Selon une caractéristique particulière de l'invention, les dérivés d'amino-acides gras insaturés de formule générale (I) répondent aux critères suivants :

• n est égal à 3.

Les dérivés d'amino-acides gras insaturés de formule générale (I), tels que définis précédemment, peuvent se présenter sous forme d'isomères Z ou E, ou d'un mélange d'isomères Z et d'isomères E, en toutes quantités.

Au sens de la présente invention, les "sels d'addition d'acides pharmaceutiquement acceptables" signifie les sels formés par addition d'un acide sur le composé, qui sont non toxiques et qui possèdent l'activité pharmacologique du composé parent.

Les sels d'addition d'acides peuvent être formés, à partir d'acides inorganiques tels que l'acide chlorhydrique, l'acide bromhydrique, l'acide sulfurique, l'acide nitrique ou l'acide phosphorique, ou bien à partir d'acides organiques tels que l'acide acétique, l'acide benzènesulfonique, l'acide benzoïque, l'acide camphresulfonique, l'acide citrique, l'acide ethane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzol-L-tartrique, l'acide tartrique, l'acide p-toluènesulfonique, l'acide triméthylacétique ou l'acide trifluoroacétique.

Les sels pharmaceutiquement acceptables préférés sont les sels formés à partir d'acide chlorhydrique.

Les composés, objet de la présente invention, se présentent de préférence sous forme de sels de chlorhydrate.

De manière avantageuse, les dérivés d'amino-acides gras insaturés de l'invention sont choisis parmi :

- le chlorhydrate de l'acide (*E*)-6-amino-hex-2-énoïque,
- le chlorhydrate de l'acide (*E*)-7-amino-hept-2-énoïque,
- le chlorhydrate de l'acide (*E*)-8-amino-oct-2-énoïque,
- le chlorhydrate de l'acide (*E*)-9-amino-non-2-énoïque,
- le chlorhydrate de l'acide (*E*)-10-amino-déc-2-énoïque,
- le chlorhydrate de l'acide (*E*)-14-amino-tétradéc-2-énoïque,
- le chlorhydrate de l'acide 6-amino-2-fluoro-hex-2-énoïque sous forme d'un mélange d'isomères Z et E,
- le chlorhydrate de l'acide (*Z*)-6-amino-2-fluoro-hex-2-énoïque,
- le chlorhydrate de l'acide (*E*)-6-amino-2-fluoro-hex-2-énoïque,
- le chlorhydrate de l'acide 7-amino-2-fluoro-hept-2-énoïque sous forme d'un mélange d'isomères Z et E,
- le chlorhydrate de l'acide (*Z*)-7-amino-2-fluoro-hept-2-énoïque,
- le chlorhydrate de l'acide 9-amino-2-fluoro-non-2-énoïque,
- le chlorhydrate de l'acide 10-amino-2-fluoro-déc-2-énoïque, et
- le chlorhydrate de l'acide 14-amino-2-fluoro-tétradéc-2-énoïque.

La présente invention a également pour objet les dérivés d'amino-acides gras insaturés de l'invention, à titre de composés chimiques nouveaux, répondant à la formule générale (I) suivante :

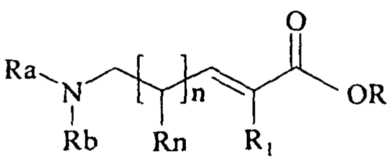

**(I)**

ainsi que leurs sels d'addition d'acides pharmaceutiquement acceptables, dans laquelle :

• Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;
• $R_1$ représente un atome d'hydrogène, de fluor, de chlore, ou de brome, ou bien un groupement $-CF_3$ ou $-CHF_2$ ou bien un groupement alkyle, alkényle ou alkynyle, linéaire ou ramifié, comprenant 1 à 6 atomes de carbone, et étant éventuellement substitué par un atome d'halogène, en particulier de fluor ;
• R représente un atome d'hydrogène ou un groupement R' représentant un groupement alkyle, linéaire ou ramifié,

comprenant 1 à 6 atomes de carbone, ou cycloalkyle comprenant 3 à 6 atomes de carbone, et étant éventuellement substitué par un atome d'halogène, en particulier de fluor ;

- Ra et Rb représentent indépendamment l'un de l'autre un atome d'hydrogène ou un groupement alkyle ou acyle, linéaire ou ramifié, comprenant 1 à 6 atomes de carbone, Ra et Rb pouvant former ensemble un cycle hydrocarboné comportant de 4 à 6 atomes de carbone ; et
- n est un entier compris entre 2 et 14 ;

à l'exclusion des composés suivants : l'acide 6-amino-hex-2-ènoïque, son chlorhydrate et son trifluoroacétate, l'acide 11-amino-2-undécènoïque, le trifluoroacétate d'acide 8-amino-oct-2-ènoïque, l'acide 8-(diméthylamino)-oct-2-ènoïque, l'acide 12-(diméthylamino)-dodéc-2-ènoïque, le 6-(isopropylamino)-2-méthyl-hex-2-énoate d'éthyle, le 6-(*tert*-butylamino)-2-méthyl-hex-2-énoate d'éthyle, le 6-amino-2-méthyl-hex-2-énoate de méthyle, le 7-amino-hept-2-énoate de méthyle, le 7-amino-2-méthyl-hept-2-énoate de méthyle et le 7-amino-4-méthyl-hept-2-énoate de méthyle.

Selon une caractéristique particulière de l'invention, les dérivés d'amino-acides gras insaturés de formule générale (I) répondent aux critères suivants :

- Rn représentent un atome d'hydrogène sur tous les carbones de la chaîne carbonée sauf sur un où Rn représente un groupement alkyle, linéaire ou ramifié, comprenant 1 à 6 atomes de carbone, et étant éventuellement substitué par un ou plusieurs atomes d'halogène, en particulier de fluor ;
- R représente un atome d'hydrogène ou un groupement alkyle, linéaire ou ramifié, comprenant 1 à 6 atomes de carbone, ou cycloalkyle comprenant 3 à 6 atomes de carbone, et étant éventuellement substitué par un atome d'halogène, en particulier de fluor ; R représente de préférence un atome d'hydrogène ;
- n, Ra, Rb et $R_1$ sont tels que définis précédemment.

Selon une caractéristique particulière de l'invention, les dérivés d'amino-acides gras insaturés de formule générale (I) répondent aux critères suivants :

- Ra et Rb représentent un atome d'hydrogène.

Selon une caractéristique particulière de l'invention, les dérivés d'amino-acides gras insaturés de formule générale (I) répondent aux critères suivants :

- R représentent un atome d'hydrogène.

Selon une caractéristique particulière de l'invention, les dérivés d'amino-acides gras insaturés de formule générale (I) répondent aux critères suivants :

- Rn représentent un atome d'hydrogène.

Selon une caractéristique particulière de l'invention, les dérivés d'amino-acides gras insaturés de formule générale (1) répondent aux critères suivants :

- $R_1$ représente un atome d'hydrogène ou de fluor.

Selon une caractéristique particulière de l'invention, les dérivés d'amino-acides gras insaturés de formule générale (1) répondent aux critères suivants :

- n est compris entre 3 et 5.

Selon une caractéristique particulière de l'invention, les dérivés d'amino-acides gras insaturés de formule générale (1) répondent aux critères suivants :

- n est compris entre 9 et 14.

Selon une caractéristique particulière de l'invention, les dérivés d'amino-acides gras insaturés de formule générale (I) répondent aux critères suivants :

- n est égal à 3.

Les dérivés d'amino-acides gras insaturés de formule générale (I), tels que définis précédemment, peuvent se présenter sous forme d'isomères Z ou E, ou d'un mélange d'isomères Z et d'isomères E, en toutes quantités.

Au sens de la présente invention, les "sels d'addition d'acides pharmaceutiquement acceptables" signifie les sels formés par addition d'un acide sur le composé, qui sont non toxiques et qui possèdent l'activité pharmacologique du composé parent.

Les sels d'addition d'acides peuvent être formés, à partir d'acides inorganiques tels que l'acide chlorhydrique, l'acide bromhydrique, l'acide sulfurique, l'acide nitrique ou l'acide phosphorique, ou bien à partir d'acides organiques tels que l'acide acétique, l'acide benzènesulfonique, l'acide benzoïque, l'acide camphresulfonique, l'acide citrique, l'acide ethane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzol-L-tartrique, l'acide tartrique, l'acide p-toluènesulfonique, l'acide triméthylacétique ou l'acide trifluoroacétique.

Les sels pharmaceutiquement acceptables préférés sont les sels formés à partir d'acide chlorhydrique.

Les composés, objet de la présente invention, se présentent de préférence sous forme de sels de chlorhydrate.

De manière avantageuse, les dérivés d'amino-acides gras insaturés de l'invention sont choisis parmi :

- le chlorhydrate de l'acide (*E*)-6-amino-hex-2-énoïque,
- le chlorhydrate de l'acide (*E*)-7-amino-hept-2-énoïque,
- le chlorhydrate de l'acide (*E*)-8-amino-oct-2-énoïque,
- le chlorhydrate de l'acide (*E*)-9-amino-non-2-énoïque,
- le chlorhydrate de l'acide (*E*)-10-amino-déc-2-énoïque,
- le chlorhydrate de l'acide (*E*)-14-amino-tétradéc-2-énoïque,
- le chlorhydrate de l'acide 6-amino-2-fluoro-hex-2-énoïque sous forme d'un mélange d'isomères Z et E,
- le chlorhydrate de l'acide (*Z*)-6-amino-2-fluoro-hex-2-énoïque,
- le chlorhydrate de l'acide (*E*)-6-amino-2-fluoro-hex-2-énoïque,
- le chlorhydrate de l'acide 7-amino-2-fluoro-hept-2-énoïque sous forme d'un mélange d'isomères Z et E,
- le chlorhydrate de l'acide (*Z*)-7-amino-2-fluoro-hept-2-énoïque,
- le chlorhydrate de l'acide 9-amino-2-fluoro-non-2-énoïque,
- le chlorhydrate de l'acide 10-amino-2-fluoro-déc-2-énoïque, et
- le chlorhydrate de l'acide 14-amino-2-fluoro-tétradéc-2-énoïque.

La présente invention a également pour objet les compositions dermatologiques comprenant à titre de principe actif au moins un dérivé d'amino-acides gras de formule générale (I), tel que défini précédemment pour les dérivés à titre de médicament, en association avec un excipient dermocosmétologiquement acceptable.

La présente invention a également pour objet l'utilisation des dérivés amino-acides gras insaturés de formule générale (I), tels que définis précédemment pour les dérivés à titre de médicament, pour la préparation d'une composition dermatologique antiradicalaire, anti-inflammatoire, anti-purigineuse, anti-collagénase, et/ou destinée au traitement du vieillissement cutané, et/ou destinée à traiter des troubles de la kératinisation et de la pigmentation et/ou destinée à améliorer la cicatrisation.

Les dérivés amino-acides gras insaturés de formule générale (I) sont plus particulièrement destinés à des compositions destinées au traitement du psoriasis, du prurit et/ou de la dermite atopique, ainsi qu'à la repigmentation des cheveux ou de la peau, notamment des taches de vieillesse blanches.

Selon la présente invention, les dérivés d'amino-acides gras insaturés de formule générale (I) sont plus particulièrement destinés à des compositions destinées à provoquer un éclaircissement de la peau ou à traiter les taches de vieillesse brunes.

En raison de leur activité anti-radicalaire, les dérivés d'amino-acides gras insaturés de formule générale (I) sont également utiles pour éviter ou limiter la photocarcinogénèse cutanée à des stades précoces et donc peuvent être utilisés dans la prévention et le traitement de diverses maladies tumorales de la peau.

La présente invention a également pour objet un procédé de préparation d'un dérivé d'aminoacide gras insaturé de formule générale (I) suivante :

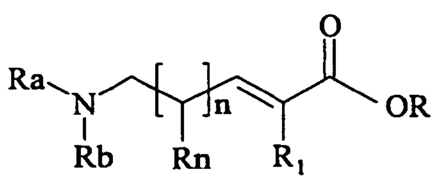

(I)

dans laquelle :

- Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;
- $R_1$ représente un atome d'hydrogène, de fluor, de chlore, ou de brome, ou bien un groupement $-CF_3$ ou $-CHF_2$ ou bien un groupement alkyle, alkényle ou alkynyle, linéaire ou ramifié, comprenant 1 à 10 atomes de carbone, et étant éventuellement substitué par un atome d'halogène, en particulier de fluor ;
- R représente un atome d'hydrogène ou un groupement R' représentant un groupement alkyle, linéaire ou ramifié, comprenant 1 à 6 atomes de carbone, ou cycloalkyle comprenant 1 à 6 atomes de carbone, et étant éventuellement substitué par un atome d'halogène, en particulier de fluor ;
- Ra et Rb représentent indépendamment l'un de l'autre un atome d'hydrogène ou un groupement alkyle ou acyle, linéaire ou ramifié, comprenant 1 à 6 atomes de carbone, Ra et Rb pouvant former ensemble un cycle hydrocarbonée comportant de 4 à 6 atomes de carbone ; et
- n est un entier compris entre 2 et 14 ;

ledit procédé de préparation comprenant :

- la mise en oeuvre de la réaction de Wittig-Horner par la réaction d'un phosphonate de formule (II) suivante :

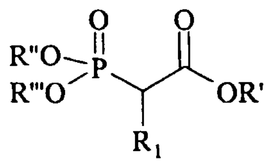

(II)

dans laquelle :

- $R_1$ est tel que défini ci-dessus,
- R' représente un groupement alkyle, linéaire ou ramifié, comprenant de 1 à 6 atomes de carbone, et est de préférence un groupement éthyle, et
- R" et R"' représentent, indépendamment l'un de l'autre, un groupe alkyle, linéaire ou ramifié, comprenant de 1 à 6 atomes de carbone, et de préférence un groupe éthyle ou méthyle, lesdits groupes R" et R"' pouvant former un cycle hydrocarboné comprenant de 2 à 4 atomes de carbone,

ou, lorsque $R_1$ = H, la mise en oeuvre de la réaction de Doebner-Knoevenagel par la réaction d'un dérivé d'acide malonique de formule $R'OOC-CH_2-COOR'$, R' étant tel que défini ci-dessus,
sur un composé de formule (III) suivante :

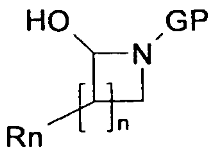

(III)

dans laquelle :

- n est tel que défini ci-dessus, et
- GP représente un groupe protecteur, N-GP représentant avantageusement un groupe de type carbamate et de préférence un carbamate de *tert*-butyle ou de benzyle,

GP correspond notamment à un groupe protecteur tel que défini dans « Protective Groups in Organic Synthesis » Third edition Theodora GREEN & Peter WUTS Wiley Interscience ISBN 0-471-16019-9 Chapitre 2 pages 17 à 246,
afin d'obtenir un composé de formule (IV) suivante :

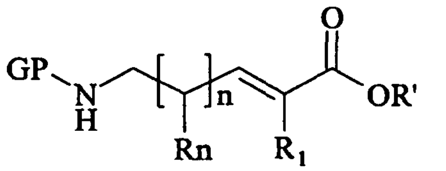

(IV)

pour lequel GP, R', $R_1$, Rn et n sont tels que définis ci-dessus,

- une éventuelle réaction de saponification du composé de formule générale (IV) susmentionnée, pour obtenir un composé de formule générale (V) suivante :

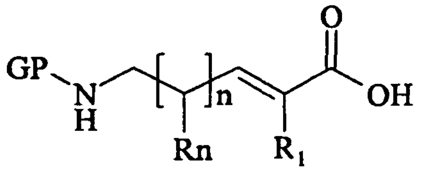

(V)

pour lequel GP, $R_1$, Rn et n sont tels que définis ci-dessus,

- une réaction de déprotection de l'azote du composé de formule (IV) ou (V) susmentionnée, pour obtenir un composé de formule (I) dans laquelle Ra et Rb représentent un hydrogène,
- et une éventuelle réaction de *N*-alkylation ou *N*-acylation du composé de formule (I) précédent dans laquelle Ra et Rb représentent un hydrogène, pour obtenir un composé de formule (I) dans laquelle au moins l'un des groupes Ra et Rb ne représente pas un hydrogène.

La réaction de Doebner-Knoevenagel, utilisée pour préparer certains composés de la présente invention, est décrite notamment dans l'article de List et al. (Adv. Synth. Catal. 2005, 347, 1558-1560) pour la synthèse d'esters (E)-$\alpha,\beta$-insaturés à partir d'aldéhydes.

Le procédé de la présente invention permet la préparation d'amino-acides gras insaturés, dans d'excellentes conditions à la fois en termes de rendement mais aussi de qualité sans trace de contamination, ledit procédé pouvant être transposé au niveau industriel.

Les dérivés d'amino-acides gras insaturés obtenus par le procédé de l'invention peuvent se trouver sous la forme de stéréoisomères Z ou E, ou sous la forme d'un mélange de ces différentes formes.

De manière avantageuse, le composé de formule générale (III) précédent :

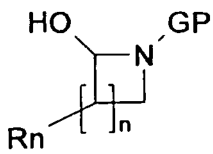

(III)

pourra être synthétisé à partir d'un composé de formule générale (VI) :

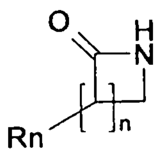

(VI)

dans laquelle n et Rn sont tels que définis précédemment,

selon les étapes suivantes :

- protection de l'azote de la fonction amide du composé de formule (VI) par un groupement GP pour obtenir un composé de formule générale (VII) suivante :

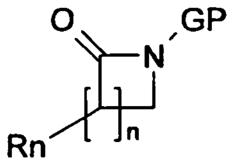

(VII)

dans laquelle n, Rn et GP sont tels que définis précédemment,
puis réduction de la fonction carbonyle du composé de formule (VII) tel que défini ci-dessus, cette étape étant notamment réalisée à l'aide d'hydrures d'aluminium tel que l'hydrure de diisobutyle aluminium à basse température.

Lorsque Ra et/ou Rb est différent d'un atome d'hydrogène, on effectue des réactions de *N*-alkylation ou *N*-acylation ou une combinaison de ces réactions selon les procédés connus de l'homme de l'art (« March's Advanced Organic Chemistry », Fifth edition, ISBN 0-471-58589-0, Michael.B. Smith and Jerry March, pages 501-552 et 511).

Les dérivés d'aminoacide gras insaturés de formule (I) dans laquelle R est différent d'un atome d'hydrogène sont obtenus de façon classique par couplage à partir de la fonction acide.

Si les composés de structure (VI) ne sont pas disponibles commercialement, il est toujours possible de les préparer à partir des cétones cycliques correspondantes selon les méthodes bien connues de l'homme de l'art, à savoir par transformation de cétones cycliques en oximes par action d'hydroxylamine, puis le traitement en milieu acide fort de ces oximes en lactame suivant une réaction très classique de Beckman (« March's Advanced Organic Chemistry », Fifth édition, ISBN 0-471-58589-0, Michael.B. Smith and Jerry March, pages 1349, 1381, 1384, 1415 - 1416) :

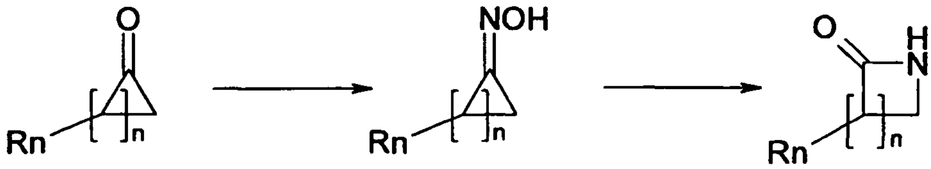

(VI)

De même, lorsque Rn est différent d'un hydrogène, il est possible de synthétiser le composé (III) à partir de l'hydroxycétone suivante :

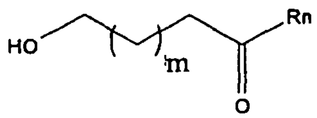

par l'enchaînement de réaction suivante : protection de la fonction alcool, réaction de Wittig-Hörner pour obtenir un ester $\alpha,\beta$-insaturé, hydrogénation de la double liaison, réduction de l'ester en aldéhyde, déprotection de la fonction alcool et réaction de Mitsunobu ou de Gabriel.

Selon un mode de réalisation particulier du procédé de l'invention, Ra et Rb représentent des hydrogènes.

Selon un autre mode de réalisation particulier du procédé de l'invention, R représente un hydrogène.

Selon encore un autre mode de réalisation particulier du procédé de l'invention, Rn représentent un hydrogène.

Selon encore un autre mode de réalisation particulier du procédé de l'invention, $R_1$ représentent un hydrogène ou un fluor.

La présente invention concerne également un procédé de préparation d'un dérivé d'aminoacide gras insaturés de formule (I-1) suivante :

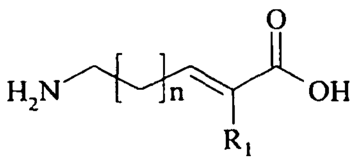

(I-1)

correspondant à un composé de formule générale (I) dans laquelle :

- Ra, Rb, Rn et R représentent un hydrogène ; et
- $R_1$ est tel que défini précédemment, c'est-à-dire qu'il représente un atome d'hydrogène, de fluor, de chlore, ou de brome, ou bien un groupement $-CF_3$ ou - $CHF_2$ ou bien un groupement alkyle, alkényle ou alkynyle, linéaire ou ramifié, comprenant 1 à 6 atomes de carbone, et étant éventuellement substitué par un atome d'halogène, en particulier de fluor ; et
- n représente un entier compris entre 2 et 14.

ledit procédé de préparation comprenant les étapes suivantes :

- une étape de protection de la fonction amide d'un composé de formule (VI-1) suivante, qui correspond à un composé de formule (VI) pour lequel Rn = H :

(VI-1)

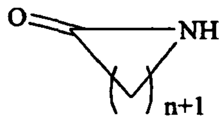

dans laquelle n est tel que défini ci-dessus dans la formule (I-1), afin d'obtenir un composé de formule (VII-1) suivante, dans laquelle la fonction amide est protégée, et correspondant à un composé de formule (VII) pour lquel Rn = H :

(VII-1)

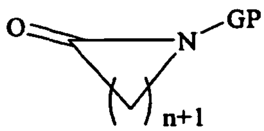

dans laquelle :

* n est tel que défini ci-dessus dans la formule (I-1), et
* GP représente un groupe protecteur, N-GP représentant avantageusement un groupe de type carbamate et de préférence un carbamate de t-butyle ou de benzyle, GP correspondant à un groupe protecteur tel que défini dans « Protective Groups in Organic Synthesis » Third edition Theodora GREEN & Peter WUTS Wiley Interscience ISBN 0-471-16019-9 Chapitre 7 pages 494 à 654,

- une étape de réduction de la fonction carbonyle du composé de formule (VII-1) telle que définie ci-dessus pour obtenir un composé de formule (III-1) suivante, correspondant à un composé de formule (III) pour lequel Rn = H :

(III-1)

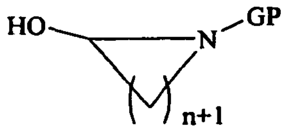

n et GP étant tels que définis ci-dessus dans la formule (VII-1),
cette étape étant notamment effectuée à l'aide de réducteurs classiques tels que les hydrures encombrés de l'aluminium comme le diisobutyle aluminium hydrure à basse température,

- la mise en oeuvre de la réaction de Wittig-Horner par la réaction d'un phosphonate de formule (II) suivante :

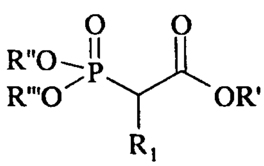

(II)

dans laquelle :

- $R_1$ est tel que défini ci-dessus,
- R' représente un groupe alkyle, linéaire ou ramifié, comprenant de 1 à 6 atomes de carbone, et est de préférence un groupe éthyle, et
- R'' et R''' représentent, indépendamment l'un de l'autre, un groupe alkyle, linéaire ou ramifié, comprenant de 1 à 6 atomes de carbone, et de préférence un groupe éthyle ou méthyle, lesdits groupes R'' et R''' pouvant former ensemble un cycle hydrocarboné comprenant de 2 à 4 atomes de carbone,

ou, lorsque $R_1$ = H, la mise en oeuvre de la réaction de Doebner-Knoevenagel par la réaction d'un dérivé d'acide malonique de formule R'OOC-$CH_2$-COOR', R' étant tel que défini ci-dessus,
sur le composé protégé de formule (III-1) susmentionné,
afin d'obtenir un composé de formule (IV-1) suivante, correspondant à un composé de formule (IV) pour lequel Rn = H :

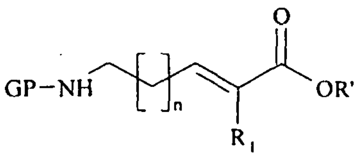

(IV-1)

dans laquelle n, GP, $R_1$ et R' sont tels que définis ci-dessus,

- une réaction de saponification du composé de formule (IV-1) susmentionnée, pour obtenir un composé de formule (V-1) suivante, correspondant à un composé de formule générale (V) pour lequel Rn = H :

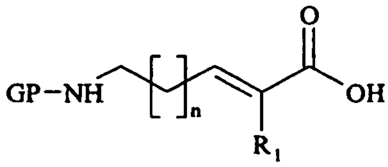

(V-1)

dans laquelle n, GP et $R_1$ sont tels que définis ci-dessus,

- et une réaction de déprotection de l'azote du composé de formule (V-1) susmentionnée, pour obtenir un composé de formule (I-1).

Les amino-acides gras insaturés objets de la présente invention peuvent ainsi être préparés grâce à la mise en oeuvre de réactions de type Wittig-Hörner à partir d'un phosphonate (fluoré ou non) ou de type Wittig à partir d'un triphényl phosphonium sur l'alpha hydroxy amine cyclisée provenant du lactame correspondant dont la fonction amine sera préalablement protégée (voir schéma de synthèse développé ci-après).

S'en suit éventuellement une réaction de saponification de l'amino-ester protégé pour obtenir un amino-acide qui sera déprotégé par une hydrolyse acide pour donner l'amino acide gras insaturé de formule générale :

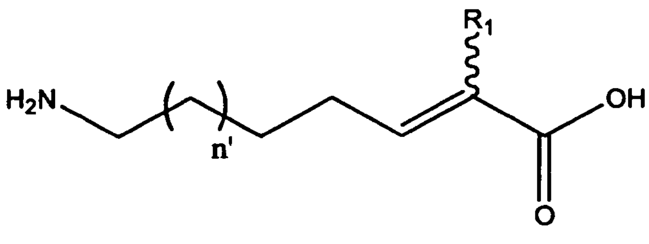

n' = n - 2 et $R_1$ = H, halogène, ou alkyle

La réaction de Wittig-Hörner est une réaction décrite dans le document Modern et toute condition expérimentale décrite dans l'état de la technique peut être utilisée dans le cadre de la présente invention. A titre d'exemple, la réaction

de Wittig-Hörner peut être effectuée en présence de triéthylphosphonoacétate et de carbonate de potassium en milieu éthanolique.

La méthode de protection de la fonction amine est une méthode classique utilisée communément pour son avantage de ne pas être hydrolysable dans les conditions basiques (conditions de déprotection de la fonction ester) et inerte vis à vis d'autres réactifs nucléophiles. (Références : T.Kunieda, T.Higuchi, Y.Abe, and M. Hirobe, Chem. Pharm. Bull., 32, 2174, 1984 ; I. Grapsas, Y.J.Cho, and S.Mobashery, J. Org. Chem., 59, 1918; 1994).

L'étape de réduction partielle du lactame est effectuée en présence d'un agent réducteur tel que le diisobutyl aluminium hydrure. En fin de réaction, les sels d'aluminium sont éliminés par formation d'un complexe soluble dans l'eau en présence de sels de Rozen (Reagents for organic synthesis, vol.I, Louis Fieser and Mary Fieser, p. 3 6).

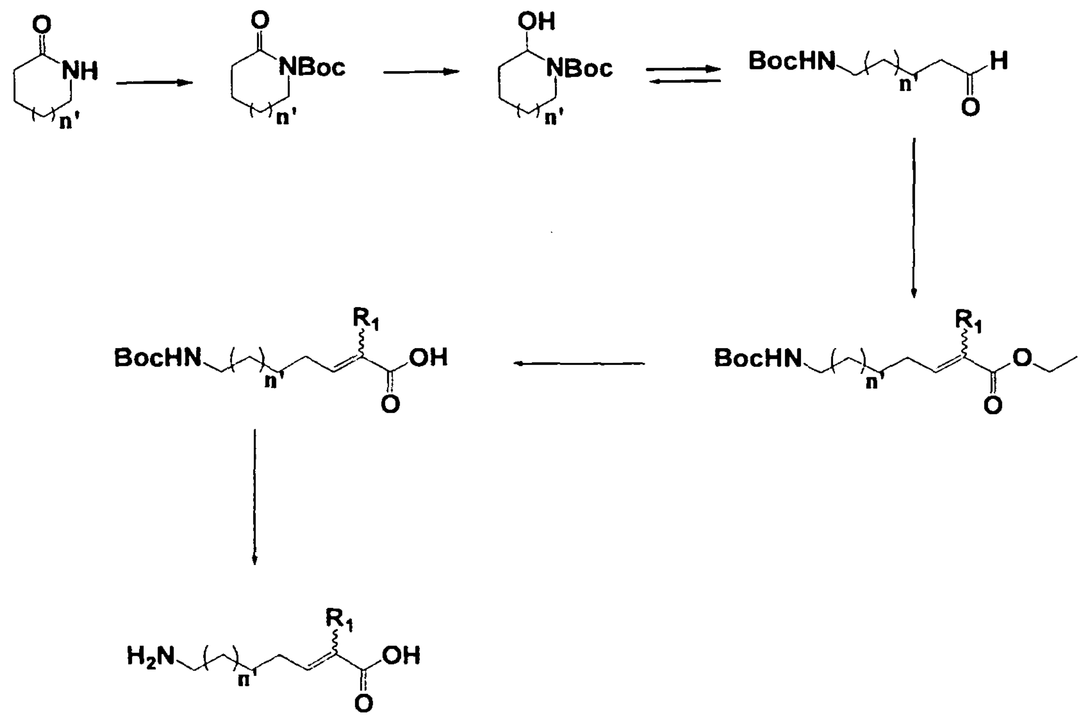

Un exemple de synthèse de dérivé où Rn=Méthyle peut être illustré par un enchaînement de réactions de protection, déprotection, Wittig-Hörner et Gabriel à partir d'une hydroxy cétone. Après protection de la fonction alcool, est réalisée une succession de réactions de Wittig Hörner. La déprotection de la fonction alcool en milieu acide, suivie d'une réaction de Gabriel génèrera la fonction amine.

La présente invention a également pour objet, à titre d'intermédiaires de synthèse, des composés de formule générale (VIII) suivante :

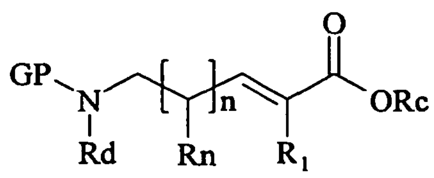

(VIII)

pour laquelle :

- Rc représente un hydrogène ou un groupement alkyle, linéaire ou ramifié, comportant de 1 à 6 atomes de carbone ;
- $R_1$ représente un atome d'hydrogène, de fluor, de chlore, ou de brome, ou bien un groupement $-CF_3$ ou $-CHF_2$ ou bien un groupement alkyle, alkényle ou alkynyle, linéaire ou ramifié, comprenant 1 à 6 atomes de carbone, et étant éventuellement substitué par un ou plusieurs atomes d'halogène, en particulier de fluor ;
- Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;
- n est un entier compris entre 2 et 14 ;

• GP représente un groupe protecteur, N-GP représentant avantageusement un groupe de type carbamate et de préférence un carbamate de tert-butyle ou de benzyle,
GP correspond notamment à un groupe protecteur tel que défini dans « Protective Groups in Organic Synthesis » Third édition Theodora GREEN & Peter WUTS Wiley Interscience ISBN 0-471-16019-9 Chapitre 2 pages 17 à 246 ; et
• Rd représente un hydrogène ;
à l'exclusion des composés suivants: le (*E*)-6-(*N*,*N*-di-*tert*-butoxy-carbonylamino)-hex-2-énoate de *tert*-butyle, le (*E*)-6-(*N*-*tert*-butoxy-carbonylamino)-hex-2-énoate de *tert*-butyle, le (*E*)-6-(*N*-*tert*-butoxy-carbonylamino)-hex-2-énoate d'éthyle, le (*E*)-7-(*N*-tert-butoxy-carbonylamino)-hept-2-énoate de méthyle et l'acide (*E*)-7-(*N*-*tert*-butoxy-carbonylamino)-hept-2-énoïque.

Selon une caractéristique particulière de l'invention, les composés de formule (VIII) répondent au critère suivant : $R_1$ représente un atome d'hydrogène, de fluor, de chlore, ou de brome, ou bien un groupement $-CF_3$ ou $-CHF_2$ ou bien un groupement alkyle, alkényle ou alkynyle, linéaire ou ramifié, comprenant 1 à 6 atomes de carbone, et étant éventuellement substitué par un ou plusieurs atomes d'halogène, en particulier de fluor.

Selon une caractéristique particulière de l'invention, les composés de formule (VIII) répondent au critère suivant : Rn représentent un hydrogène.

Selon une autre caractéristique particulière de l'invention, les composés de formule (VIII) répondent au critère suivant : $R_1$ représente un hydrogène ou un fluor.

La présente invention sera illustrée dans le cadre des exemples de synthèse donnés ci-après :

1) synthèse des composés selon l'invention

1.1) Composé n° 12 (acide (*E*)-10-amino-déc-2-énoïque)

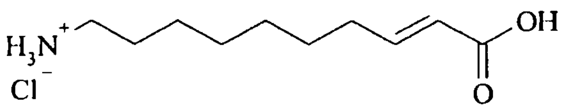

12

Stade 1

Dans un tricol sous flux d'azote, 10.0g de Caprylolactame (70.8mmol) sont solubilisés dans 150ml de Tétrahydrofurane. 10.06ml (1.01 eq) de Triéthylamine sont additionnés ainsi que 8.74g (1.01 eq) de 4-Diméthylaminopyridine, et enfin 30.91g (2 eq) de di-terButyl di-carbonate. Le milieu réactionnel est agité toute la nuit à température ambiante. Un suivi CCM permet de contrôler la fin de la réaction. Le milieu réactionnel est concentré, repris par de l'eau et de l'acétate d'éthyle. Le mélange est extrait par trois fois avec de l'acétate d'éthyle, les phases organiques rassemblées sont lavées par une solution d'acide chlorhydrique 5%, puis par une solution saturée de NaCl. Les phases organiques sont séchées sur $Na_2SO_4$, filtrées et concentrées sous vide, pour conduire à une huile rouge. Caractérisations :
m= 24.0g 100% rdt
$R_f$= 0.8 (DCM / MeOH 99/1)
RMN ($^1H$, $CDCl_3$): 1.50 (s, 9H); 1.53 (m, 6H); 1.67 (m, 2H); 1.82 (m, 2H); 2.87 (m, 2H), 2.78 (t, 2H).

Stade 2

Dans un tricol sous flux d'azote, 17.1g de Caprylolactame protégé par un groupement Boc (70.8mmol) sont solubilisés dans 170ml de toluène. Le milieu est refroidi à -78°C et 59.2 ml d'une solution de Dibal-H à 20% dans le toluène (1.01 eq) sont coulés goutte à goutte, sur 1 heure, en maintenant la température du milieu entre -80°C et -75°C. Un suivi CCM permet de contrôler la fin de la réaction. A -78°C, 480ml d'une solution saturée de tartrate double sont coulés lentement et le milieu est agité vigoureusement pendant une nuit. La phase organique est extraite. La phase aqueuse est extraite à l'acétate d'éthyle par trois fois. Les phases organiques rassemblées sont lavées par une solution saturée de NaCl, séchées sur $MgSO_4$, filtrées et concentrées sous vide, pour conduire à un solide orange.
Caractérisations :
m= 20.0g 99% rdt
$R_f$= 0.1 (Heptane / Acétate d'éthyle 7/3)

RMN ($^1H$, $CDCl_3$) : 1.40-1.80 (m, 20H) ; 2.85-2.89 (m, 2H) ; 3.75-3.79 (m, 2H).

Stade 3

Dans un tricol sous flux d'azote, 2.0g (8.22mmol) de composé du stade 2 sont solubilisés dans 20ml d'éthanol. 1.70g (1.5eq) de carbonate de potassium sont ajoutés au milieu et 1.96ml (1.2eq) de Triéthylphosphonoacétate sont coulés lentement. Le mélange est alors chauffé à 45°C pour une nuit. Un suivi CCM permet de contrôler la fin de la réaction. Le milieu réactionnel est concentré puis le résidu est repris par de l'acétate d'éthyle et de l'eau. La phase aqueuse est extraite par de l'acétate d'éthyle par trois fois. Les phases organiques rassemblées sont lavées par une solution saturée de NaCl, séchées sur $MgSO_4$ filtrées et concentrées sous vide. Le produit brut obtenu est purifié par chromatographie sur gel de silice (gradient heptane/ AcOEt), pour conduire à une huile jaune.
Caractérisations :
m= 1.60g 62% rdt
$R_f$= 0.4 (Heptane / Acétate d'éthyle 7/3)
RMN ($^1H$, $CDCl_3$, 300 MHz) : δ 1.28-1.46 (m, 22H) ; 2.16-2.19 (m, 2H) ; 3.10 (m, 2H) ; 4.20 (q, 2H) ; 5.82 (dt, 1H, *J*=15.6Hz) ; 6.97 (dt, 1H, *J*=15.6Hz).

Stade 4

Dans un ballon, 1.60g (5.10mmol) de composé du stade 3 sont mis en solution dans 16ml de THF. 6.40ml (2.5eq) d'une solution de NaOH 2M sont ajoutés et le milieu réactionnel est chauffé à 65°C pendant une nuit. Un suivi CCM permet de contrôler la fin de la réaction. Le pH du mélange est alors ajusté à 4 par ajout d'acide chlorhydrique. Le milieu est concentré, repris par AcOEt / eau. La phase aqueuse est extraite par de l'acétate d'éthyle (trois fois) ; les phases organiques rassemblées sont lavées par une solution saturée de NaCl, séchées sur $Na_2SO_4$, filtrées et concentrées sous vide, pour conduire à un solide beige.
Caractérisations :
m=1.2g 82% rdt
$R_f$ = 0.2 (Heptane / Acétate d'éthyle 7/3)
RMN H, $CDCl_3$, 300 MHz) : δ 1.30-1.46 (m, 19H) ; 2.20-2.27 (m, 2H) ; 3.13 (m, 2H) ; 5.80-5.86 (dt, 1H, *J*=15.6Hz) ; 7.0 (dt, 1H, *J*=15.6Hz).

Stade 5

Dans un ballon sous baudruche d'azote, 1.2g (4.28mmol) de composé du stade 4 sont solubilisés dans 7.5ml (3.5eq) d'une solution de HCl dans l'éther 2M. Le milieu est agité toute une nuit à 35°C. Le suivi CCM permet de contrôler la fin de la réaction. Le milieu est concentré sous vide, repris dans du dichlorométhane, filtré et lavé par du dichlorométhane, séché sous vide, pour conduire à un solide blanc. Ce solide est plusieurs fois recristallisé dans l'éthanol.
Caractérisations
m= 0.59g 82% rdt
$R_f$ = 0.1 (Heptane / Acétate d'éthyle 5/5)
RMN ($^1H$, MeOD, 300 MHz) : δ 1.50-1.69 (m, 10H) ; 2.21-2.28 (q, 2H) ; 2.93 (t, 2H) ; 5.80 (d, 1H, *J* = 15.6Hz) ; 6.95 (dt, 1H, *J* = 15.6Hz)
Spectrométrie de masse : $[M+H]^+$ = 186 (calculé 185)

1.2) Composé n° 13 (acide 10-amino-2-fluoro-déc-2-énoïque)

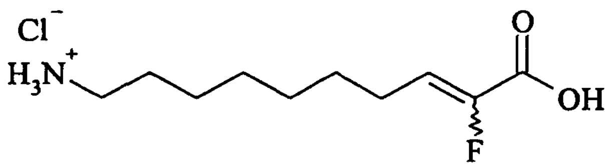

13

Les stades 1 et 2 sont conduits de la même façon que précédemment, sur le Caprylolactame.

Stade 3

Dans un tricol sous flux d'azote, 2.0g (8.22mmol) de composé du stade 2 sont solubilisés dans 20ml d'éthanol. 1.70g (1.5eq) de carbonate de potassium sont ajoutés au milieu et 2.06g (1.1eq) de Triéthylphosphonofluoroacétate sont coulés lentement. Le mélange est alors chauffé à 45°C pour une nuit. Un suivi CCM permet de contrôler la fin de la réaction. Le milieu réactionnel est concentré puis le résidu est repris par de l'acétate d'éthyle et de l'eau. La phase aqueuse est extraite par de l'acétate d'éthyle par trois fois. Les phases organiques rassemblées sont lavées par une solution saturée de NaCl, séchées sur $MgSO_4$ filtrées et concentrées sous vide. Le produit brut obtenu est purifié par chromatographie sur gel de silice (gradient heptane/ AcOEt), pour conduire à une huile jaune pâle.
Caractérisations :
m= 1.70g 65% rdt
$R_f$ = 0.5 (Heptane / Acétate d'éthyle 7/3)
RMN ($^1$H, $CDCl_3$, 300MHz): δ 1.32-1.39 (m, 22H) ; 2.21 (m, 2H) ; 3.11 (m, 2H) ; 4.30 (q, 2H) ; 5.89-5.96 (dt, 0.5H, *J*=21.9Hz, forme E) ; 6.06-6.18 (dt, 0.5H, *J*=33.3Hz, forme Z).

Les stades 4 et 5 sont conduits comme précédemment et conduisent aux caractérisations suivantes :
Caractérisations stade 4 :
m=1.4g 87% rdt
$R_f$ = 0.2 (Heptane / Acétate d'éthyle 7/3)
RMN ($^1$H, $CDCl_3$) : δ□ 1.23-1.46 (m, 19H) ; 2.26-2.55 (m, 2H) ; 3.12 (m, 2H), 5.98-6.32 (ddt, 1H, forme E et Z).
Caractérisations stade 5 :
m= 0.9g 95% rdt
$R_f$ = 0.1 (Heptane / Acétate d'éthyle 5/5)
RMN (1H, MeOD) : δ □1.41-1.53 (m, 8H) ; 1.65-1.70 (m, 2H), 2.24-2.29 (m, 1H forme Z) ; 2.53-2.56 (m, 1H, forme E) ; 2.91-2.96 (t, 2H), 5.90-6.03 (dt, 0.5H, J=21.6Hz, forme E) ; 6.08-6.25 (dt, 0.5H, J=33.3Hz, forme Z).
Spectrométrie de masse : $[M+H]^+$ = 204 (calculé 203)

1.3) Composé n° 7 (acide (*E*)-8-amino-oct-2-énoïque)

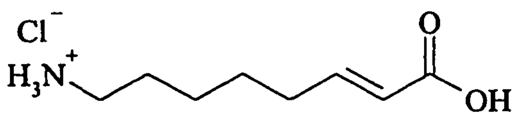

**7**

Le protocole général utilisé pour la préparation du composé 12 est appliqué à 1'ε-caprolactame, pour conduire à l'acide *(E)*-8-amino-oct-2-ènoïque.
$R_f$ = 0.1 (Heptane / Acétate d'éthyle 5/5)
RMN ($^1$H, MeOD) : δ1.41-1.57 (m, 4H) ; 1.67-1.75 (m, 2H) ; 2.24-2.31 (m, 2H) ; 2.93-2.98 (m, 2H) ; 5.86 (dd, 1H) ; 6.98 (dt, 1H).
Spectrométrie de masse : $[M+H]^+$ = 158 (calculé 157)

1.4) Composé n°8 (acide *(E)*-9-amino-non-2-ènoïque)

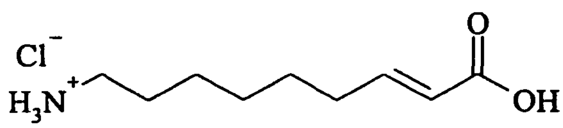

**8**

Le protocole général utilisé pour la préparation du composé 12 est appliqué à l'oenantholactame pour conduire à l'acide *(E)*-9-amino-non-2-ènoïque.
$R_f$ = 0.1 (Heptane / Acétate d'éthyle 5/5)

RMN ($^1$H, DMSO-d6) : δ □1.29-1.54 (m, 8H) ; 2.16-2.23 (m, 2H) ; 2.71-2.76 (m, 2H) ; 5.77 (dd, 1H, *J*=18Hz) ; 6.81 (dt, 1H, *J*=18Hz).
Spectrométrie de masse : $[M+H]^+$ = 172 (calculé 171)
Point de fusion: 104°C

1.5) Composé n° 9 (acide 9-amino-2-fluoro-non-2-ènoïque)

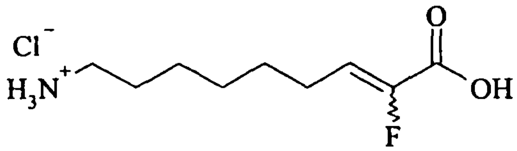

9

Le protocole général utilisé pour la préparation du composé 13 est appliqué à l'oenantholactame pour conduire à l'acide 9-amino-2-fluoro-non-2-ènoïque.
$R_f$ = 0.1 (Heptane / Acétate d'éthyle 5/5)
RMN ($^1$H, MeOD) : δ□1.40-1.71 (m, 8H) ; 2.30-2.35 (m, 2H) ; 2.91-2.96 (m, 2H) ; 5.92-6.05 (dt, 0.5H, J=21.9Hz, forme E) ; 6.09-6.26 (dt, 0.5H, J=33.3Hz, forme Z).
Spectrométrie de masse : $[M-H]^-$ = 188 (calculé 189)
Point de fusion: 120°C

1.6) Composé n° 11 (acide (E)-14-amino-tétradéc-2-ènoïque)

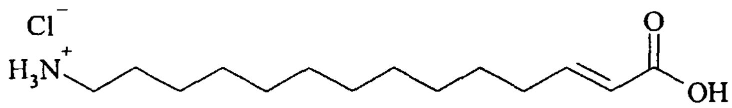

11

Le protocole général utilisé pour la préparation du composé 12 est appliqué à l'azacyclodécanone pour conduire à l'acide (E)-14-amino-tétradéc-2-ènoïque.
$R_f$ = 0.1 (Heptane / Acétate d'éthyle 5/5)
RMN ($^1$H, MeOD) : ε□1.31-1.69 (m, 18H) ; 2.20-2.27 (m, 2H) ; 2.90-2.95 (m, 2H) ; 5.80 (dd, 1H, *J*=15.6Hz) ; 6.96 (dt, 1H, *J*=15.6Hz).
Spectrométrie de masse : $[M+H]^+$ = 242 (calculé 241)
Point de fusion: 153°C

1.7) Composé n° 10 (acide 14-amino-2-fluoro-tétradéc-2-ènoïque)

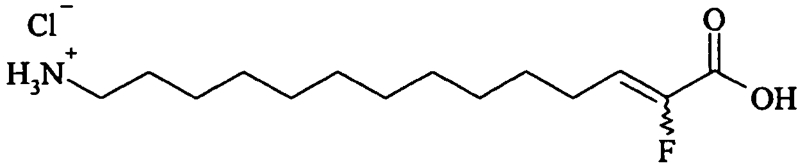

10

Le protocole général utilisé pour la préparation du composé 13 est appliqué à l'azacyclodécanone pour conduire

à l'acide 14-amino-2-fluoro-tétradéc-2-ènoïque.
$R_f$=0.1 (Heptane / Acétate d'éthyle 5/5)
RMN ($^1H$, MeOD) : δ□1.34-1.67 (m, 18H) ; 2.21-2.26 (m, 1H forme Z) ; 2.48-2.54 (m, 1H, forme E) ; 2.92 (t, 2H), 5.91-6.04 (dt, 0.5H, J=21.9Hz, forme E) ; 6.09-6.25 (dt, 0.5H, J=33.3Hz, forme Z).
Spectrométrie de masse : $[M+H]^+$ = 260 (calculé 259)
Point de fusion : 148.5°C

1.8) Composé n° 1 (acide (*E*)-6-amino-hex-2-ènoïque)

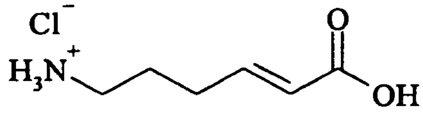

1

Le protocole général utilisé pour la préparation du composé 12 est appliqué à la 2-pyrrolidone pour conduire à l'acide *(E)*-6-amino-hex-2-ènoïque.
$R_f$=0.1 (Heptane / Acétate d'éthyle 5/5)
RMN ($^1H$, MeOD) : δ□ 1.81-1.91 (m, 2H) ; 2.32-2.40 (m, 2H) ; 2.95-3.00 (m, 2H) ; 5.90 (dd, 1H, *J*=15.6Hz) ; 6.96 (dt, 1H, *J*=15.6Hz).
Spectrométrie de masse : $[M+H]^+$ = 130 (calculé 129)

1.9) Composé n° 2 (acide *(E)*-6-amino-2-fluoro-hex-2-ènoïque) et Composé n° 3 (acide *(Z)*-6-amino-2-fluoro-hex-2-ènoïque)

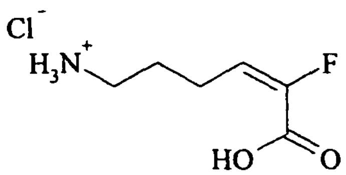

2

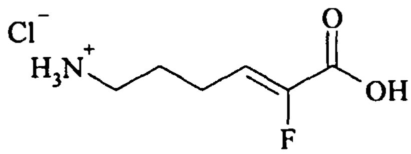

3

Le protocole général utilisé pour la préparation du composé 13 est appliqué à la 2-pyrrolidone pour conduire à l'acide *(Z)*-6-amino-2-fluoro-hex-2-ènoïque (n° 3) et à l'acide *(E)*-6-amino-2-fluoro-hex-2-ènoïque (n° 2), qui ont été dans ce cas séparés.
Forme Z
$R_f$=0.1 (Heptane / Acétate d'éthyle 5/5)
RMN ($^1H$, MeOD) : δ□ 1.80-1.90 (m, 2H) ; 2.33-2.42 (m, 2H) ; 2.95-3.00 (m, 2H) ; 6.12-6.28 (dt, 1H, J=32.7Hz, forme Z).
Spectrométrie de masse : $[M+H]^+$ = 148 (calculé 147)
Forme E
$R_f$=0.1 (Heptane / Acétate d'éthyle 5/5)
RMN ($^1H$, MeOD) : δ 1.77-1.89 (m, 2H); 2.60-2.68 (m, 2H); 2.94-2.99 (m, 2H) ; 5.96-6.08 (dt, 1H, J=21.0Hz, forme E).
Spectrométrie de masse : $[M+H]^+$ = 148 (calculé 147)

1.10) Composé n° 4 (acide *(E)*-7-amino-hept-2-ènoïque)

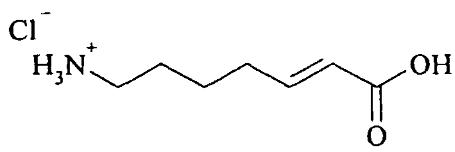

4

Le protocole général utilisé pour la préparation du composé 12 est appliqué au δ-valérolactame pour conduire à l'acide *(E)*-7-amino-hept-2-ènoïque.
$R_f$ = 0.1 (Heptane / Acétate d'éthyle 5/5)
RMN ($^1$H, MeOD) : δ □1.56-1.74 (m, 4H) ; 2.28-2.34 (m, 2H) ; 2.94-2.99 (m, 2H) ; 5.84 (dd. 1H, *J*=15.6Hz) ; 6.97 (dt, 1H, *J*=15.6Hz).
Spectrométrie de masse : $[M+H]^+$ = 144 (calculé 143)

1.11) Composé n° 5 (acide *(Z+E)*-7-amino-2-fluoro-hept-2-ènoïque) et Composé n° 6 (acide *(Z)*-7-amino-2-fluoro-hept-2-ènoïque)

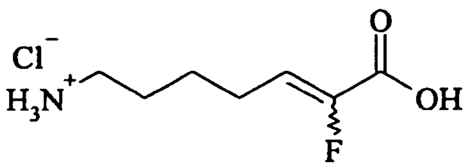

5

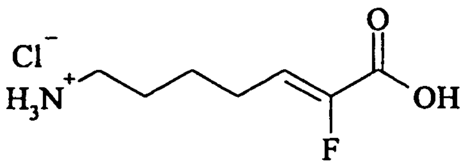

6

Le protocole général utilisé pour la préparation du composé 13 est appliqué au δ-valérolactame pour conduire à l'acide *(Z)*-7-amino-2-fluoro-hept-2-ènoïque (n° 6) et au mélange d'acide *(Z+E)*-7-amino-2-fluoro-hept-2-ènoïque (n° 5).
Forme Z
$R_f$ = 0.1 (Heptane / Acétate d'éthyle 5/5)
RMN ($^1$H, MeOD) : δ □1.55-1.71 (m, 4H) ; 2.32-2.35 (m, 2H) ; 2.93-2.98 (m, 2H) ; 6.11-6.28 (dt, 1H, J=33.3Hz, forme Z).
Spectrométrie de masse : $[M+H]^+$ = 162 (calculé 161)
Forme E +Z
$R_f$ = 0.1 (Heptane / Acétate d'éthyle 5/5)
RMN ($^1$H, MeOD) : δ□ 1.54-1.75 (m, 4H) ; 2.30-2.41 (m, 0.6H) ; 2.56-2.64 (m, 1.4H, forme E) ; 2.94-2.99 (m, 2H) ; 5.95-6.08 (dt, 0.7H, *J*=21.6Hz, forme E) ; 6.12-6.28 (dt, 0.3H, *J*=33.3Hz, forme Z).
Spectrométrie de masse : $[M+H]^+$ = 162 (calculé 161).

2) Effet anti-inflammatoire analysé au niveau des médiateurs lipidiques de l'inflammation

Le kératinocyte, cellule la plus représentée au niveau de l'épiderme, libère, en réponse à de nombreux facteurs extra-cellulaires présents dans son environnement, des médiateurs biologiquement actifs, notamment des prostaglandines et les leucotrienes qui jouent un rôle important dans l'initiation et la modulation des réactions inflammatoires cutanées et qui interviennent également dans la régulation de la réponse immune. La prostaglandine PG6KFlalpha, est un des métabolites majeurs produits par le kératinocyte stimulé, et représentatif de la modulation de la production des métabolites du métabolisme de l'acide arachidonique issus de la voie de la cyclo-oxygénase.

2.1) PROTOCOLE:

La suspension de kératinocytes dans le DMEM 10% SVF est distribuée dans des plaques 6 puits (1,2 106 cellules/puits), et incubée pendant 16 heures à 37°C dans une atmosphère à 5% CO2. Les kératinocytes sont alors rincés avec du PBS pour éliminer les cellules non-adhérentes puis exposés aux produits à tester inclus dans du DMEM sans SVF (qui pourrait interférer dans le dosage).
La concentration testée en culture est de 3 μg/ml. Elle a été retenue après un test préliminaire d'évaluation de la cytotoxicité (rouge neutre) et n'est pas cytotoxique.

Pour chaque traitement, 3 puits sont réalisés. Les cellules sont pré-incubées pendant 60 mn avec les produits à tester puis un agent stimulant de la cascade de l'acide arachidonique, le ionophore calcique, est ajouté pendant 5 heures: le ionophore calcique A23187 est utilisé à la concentration 1 $\mu$M.
Après ces 5 heures de culture, les milieux de culture de chacun des puits sont récupérés, centrifugés à 3000 tr/mn et conservés à -80°C.
La production de prostaglandine 6KFI$\alpha$ pour chacun des essais est mesurée par Kit Elisa EUROMEDEX.

2.2) RESULTATS :

Les résultats sont rassemblés dans le tableau 1 et sont exprimés en pourcentage d'activité / contrôle stimulé.

Tableau 1

| Composés de formule I | | | | | | % d'activité inhibitrice des molécules évaluées sur la production de PG6KF 1 $\alpha$ |
|---|---|---|---|---|---|---|
| N° | n | R | $R_n$ | $R_1$ | Sel | |
| 1 | 2 | H | H | H | HCl | + |
| 2 | 2 | H | H | F | HCl | ~ |
| 3 | 2 | H | H | F | HCl | 22 |
| 4 | 3 | H | H | H | HCl | 18 |
| 5 | 3 | H | H | F | HCl | 41 |
| 6 | 3 | H | H | F | HCl | 25 |
| 7 | 4 | H | H | H | HCl | 36 |
| 8 | 5 | H | H | H | HCl | 8 |
| 9 | 5 | H | H | F | HCl | 17 |
| 10 | 10 | H | H | F | HCl | 38 |
| 11 | 10 | H | H | H | HCl | 40 |

La synthèse des données obtenues permet de mettre en évidence les potentialités anti-inflammatoires en particulier des composés n° 11, 5, 7 et 10, avec une dose efficace 45 (DE45) pour le composé n°11 de 10$\mu$g/ml et 25$\mu$g/ml pour le composé n° 5.

<u>3) Propriété anti-inflammatoire : inhibition de la synthèse d'une cytokine pro-inflammatoire IL8</u>

La fonction barrière de la peau permet d'assurer une protection vis à vis de l'environnement extérieur, et les kératinocytes de l'épiderme peuvent directement répondre à une large variété d'agents irritants ou allergènes et participer activement aux processus inflammatoires et immunitaires cutanés, en particulier à travers la génération de cytokines pro-inflammatoires, médiateurs d'origine protéique. Parmi ces molécules biologiquement actives, l'IL1a (Interleukine 1 $\alpha$) et le TNF$\alpha$ (Tumor Necrosis Factor $\alpha$) sont considérées comme des cytokines primaires, leur libération étant suffisante pour induire l'inflammation en vertu de leur induction de molécules d'adhésion au niveau des cellules endothéliales et de leur induction de facteurs chimiotactiques tels que les chemokines. Le système des chemokines contrôle le trafic leucocytaire pendant la réponse inflammatoire et est nécessaire aux interactions des réponses immunes innées et adaptatives.
Dans l'étude présente nous nous sommes plus spécifiquement intéressés à la chemokine - Interleukine 8 - qui est fortement impliquée dans l'amplification de la réponse inflammatoire et dont la fonction principale est de recruter et activer les polynucléaires neutrophiles notamment en stimulant leur libération de molécules pro-inflammatoires.
Dans cette étude, réalisée sur des plaques 96 puits, nous avons évalué l'activité des acides amino alcènes, sur la production d'interleukine 8 induite au niveau du kératinocyte par le phorbol ester PMA et le ionophore calcique A23187.

3.1) PROTOCOLE:

La suspension de kératinocytes dans le KSFM complémenté est distribuée dans des plaques 96 puits (3.10$^4$

cellules/puits), et incubée pendant 16 heures à 37°C dans une atmosphère à 5% $CO_2$. Les kératinocytes sont alors rincés avec du PBS pour éliminer les cellules non-adhérentes puis exposés aux produits à tester inclus dans du KSFM non complémenté (qui pourrait interférer dans le dosage).

La concentration testée en culture est de 3µg/ml. Elle a été retenue après un test préliminaire d'évaluation de la cytotoxicité (rouge neutre) et n'est pas cytotoxique.

Pour chaque traitement, 3 puits sont réalisés. Les cellules sont pré-incubées pendant 60 mn avec les produits à tester puis stimulées, parallèlement à des contrôles négatifs sans stimulant : Phorbol Myristate Acétate (PMA) 1 µM + Ionophore calcique (A23187) 0,1 µM.

Incubation pendant 6 heures à 37°C en atmosphère humide d'air contenant 5% de $CO_2$.

Les milieux de culture de chacun des puits sont récupérés, centrifugés à 3000 tr/mn et conservés à -80°C.

Dosage des cytokines : l'IL8 est dosée par une méthode immunoenzymatique en kit ELISA (Immunotech).

3.2) RESULTATS :

Les résultats sont rassemblés dans le tableau 2 et sont exprimés en pourcentage d'activité / contrôle stimulé.

Tableau 2

| Composés de formule I | | | | | | % d'activité inhibitrice des molécules évaluées sur la production d'IL8 |
|---|---|---|---|---|---|---|
| N° | n | R | $R_n$ | $R_1$ | Sel | |
| 1 | 2 | H | H | H | HCl | ~ |
| | | | | F | HCl | ~ |
| 3 | 2 | H | H | F | HCl | ~ |
| 4 | 3 | H | H | H | HCl | ~ |
| 5 | 3 | H | H | F | HCl | + |
| 6 | 3 | H | H | F | HCl | + |
| 7 | 4 | H | H | H | HCl | ~ |
| 8 | 5 | H | H | H | HCl | 14 |
| 9 | 5 | H | H | F | HCl | 20 |
| 10 | 10 | H | H | F | HCl | 8 |
| 11 | 10 | H | H | H | HCl | 22 |

La synthèse des données obtenues permet de mettre en évidence les potentialités anti-inflammatoires en particulier du composé n° 11 et 9, avec une dose efficace 50 (DE 50) de 11 µg/ml pour le composé n° 11.

4) <u>Etude de l'effet anti-radicalaire sur espèces-actives de l'oxygène (EAO)</u>

Durant le métabolisme cellulaire normal, lors d'exposition occasionnelle de la peau à des agents stressants ou au cours de pathologies dermatologiques, des espèces oxygénées réactives encore appelées Espèces Activées de l'Oxygène (EAO) sont générées (Y. M. W. Janssen *et al,* 1993). Ces EAO, décrites comme des métabolites très réactifs, jouent un rôle important dans bon nombre de processus tels que l'inflammation, le vieillissement et la promotion tumorale.

Les EAO sont considérées comme les « seconds messagers » dans la signalisation cellulaire du stress oxydatif et donc comme les médiateurs précoces de l'inflammation (A. Van Der Vliet and A. Bast, 1992).

Leur surproduction induit d'importants dommages au sein de la cellule. Certains constituants cellulaires sont alors les cibles majeures d'un tel stress oxydatif : les composants lipidiques de la membrane plasmique (lipoperoxydation), les protéines (dénaturation et dégradation) et le matériel génétique ou ADN (mutations) peuvent être altérés. Les cellules sont capables de limiter ces dommages oxydatifs grâce à différents systèmes de défense antiradicalaire (antioxydants enzymatiques et non enzymatiques) (B. P. Yu, 1994 ; H. Steiling *et al,* 1999).

Cependant, dans certaines conditions, les EAO sont produites en quantité telle que l'activité antioxydante cellulaire est insuffisante ; ces EAO deviennent alors des facteurs inducteurs de pathologies inflammatoires et du vieillissement tissulaire (Y. Miyachi *et al,* 1986 ; M. Kress *et al,* 1995).

Il existe différents agents chimiques (ex : $H_2O_2$) ou physiques (ex : UVA) capables de générer *in vitro* un stress

oxydatif. Ainsi les EAO produites vont altérer diverses cibles cellulaires (Membranes, ADN ou Protéines) dont l'altération peut-être analysée par des méthodologies biochimiques largement utilisées comme le dosage des TBARS pour la lipoperoxydation lipidique, ou le dosage *in vitro* des EAO intracellulaires à l'aide de la sonde $H_2$DCF-DA.

Nous avons mis en place un modèle d'étude *in vitro* du stress oxydatif induit par $H_2O_2$ / Fer, $H_2O_2$ qui génère massivement des EAO intracellulaires par une réaction en chaîne déclenchée par l'oxydation des lipides membranaires.

Cette technique est basée sur l'utilisation d'une sonde fluorescente, la 6-carboxy-2',7'-dichlorodihydro fluorescéine diacétate, di (acétoxyméthyle ester) ($H_2$DCF-DA), qui, une fois qu'elle a pénétré dans la cellule, est désacétylée par les estérases intracellulaires formant alors du $H_2$DCF. Ce produit est oxydé par les EAO intracellulaires en un composé hautement fluorescent: la 2',7'-dichlorofluorescein (DCF) *(*Suematsu M et al., 1996, Free Radicals Pratical Approach, Punchard ed. p83-99*).*

Les matériel et méthodes utilisés pour le dosage *in vitro* des EAO intracellulaires sont indiqués ci-après.

a) Outil cellulaire:
Lignée cellulaire de fibroblastes murins cutanés L929.
b) Matériel:

- Microplaque 96 puits à fond plat
- Cytofluorimètre Cytofluor II : Réf. PERSEPTIVE BIOSYSTEMS c) Réactifs :

Réactifs de culture cellulaire :

- Milieu de culture Dulbecco's Modified Eagle Medium (DMEM)
- Sérum de veau foetal (SVF)
- Tampon phosphate PBS pH 7,4
- Trypsine EDTA (six)

Sonde fluorescente :

- 6-carboxy-2',7'-dichlorodihydrofluorescein diacétate, di(acétoxyméthyle ester) (PM. 675,43)

Produits de stimulation des cellules :

- Peroxyde d'Hydrogène ($H_2O_2$) 3% : Réf. GIFRER (Laboratoire Gifrer Barbezat)
- Ferrous Ammonium Sulfate ($Fe^{2+}$)
- Ferric Ammonium Sulfate ($Fe^{3+}$)

d) Produits testés :

Les concentrations testées sont des concentrations non cytotoxiques. La cytotoxicité a été réalisée par la méthode du rouge neutre, après incubation du produit durant 3 heures.

Le produit anti-radicalaire de référence est la vitamine E ou $\alpha$-tocophérol (PM : 430.7) (SIGMA, réf: T-1539).

La solution mère est préparée à 400mg/ml dans du DMSO et conservée à -20°C. La solution de pré-traitement est préparée extemporanément à 400$\mu$g/ml dans du milieu de culture sans SVF.

Pour l'évaluation des dérivés d'amino-acides gras insaturés, les dilutions sont préparées dans du milieu de culture extemporanément, pour une gamme de concentrations de 0.02, 0.2, 2 et 20 ng/ml.

e) Protocole :

Ensemencement des cellules :

Les cellules de la lignée de fibroblastes L929 sont ensemencées dans des microplaques de 96 puits à fond plat dans 100 $\mu$l de DMEM supplémenté de 10% de SVF, puis elles sont incubées une nuit à 37°C en atmosphère

humide à 5% de $CO_2$.

Le blanc de plaque sans cellules est évalué sur 6 puits.

Pré-traitement des cellules :

Les dilutions des produits à tester et molécule de référence sont réalisées dans le milieux de culture sans SVF, puis déposées dans 7 puits à raison de 100 μl par puits.

Les cellules sont alors incubées pendant 3 heures à 37°C en atmosphère humide à 5% de $CO_2$.

Les cellules « Témoin » (fluorescence naturelle des cellules), « Contrôle » (production basale de EAO) et « Stimulées » (production de EAO après traitement oxydatif) sont recouvertes avec 100μl de DMEM.

Les cellules « Contrôle » sont incubées avec la sonde mais ne sont ni prétraitées ni traitées.

Les cellules « Stimulées » sont incubées avec la sonde et sont traitées mais pas prétraitées.

Les cellules « Témoin » ne sont ni pré-traitées, ni incubées avec la sonde, ni traitées.

Incubation des cellules avec la sonde et stress oxydatif :

Les cellules sont rincées au PBS 1X à raison de 100μl par puits. Puis elles sont mises à incuber 30 minutes à 37°C en atmosphère humide à 5% de $CO_2$ avec 50μl de sonde $H_2$DCF-DA à 5 μM.

Après 30 minutes au contact de la sonde seule, les cellules sont mises à incuber 30 minutes à 37°C en atmosphère humide à 5% de $CO_2$ avec un ajout de 25μl d'$H_2O_2$ à 800μM et 25μl de solution de fer ferreux et ferrique à 8mM, pour avoir des concentrations finales de 200μM d'$H_2O_2$ et 2mM de fer ferreux et ferrique.

Les cellules sont ensuite rincées au PBS 1X à raison de 100μl par puits, puis incubées 30 minutes à 37°C en atmosphère humide à 5% de $CO_2$ avec 100μl de PBS 1X.

Ces 1 h 30 minutes d'incubation à 37°C permettent aux estérases intracellulaires de désacétyler la sonde en $H_2$DCF, oxydable par les EAO intracellulaire en DCF : composé fluorescent dont la formation est proportionnelle à la quantité d'EAO intracellulaires.

L'intensité de fluorescence est lue au cytofluorimètre à $\lambda_{excitation}$= 485nm et $\lambda_{émission}$= 530nm. Elle reflète la quantité d'EAO intracellulaires produite.

Schéma du protocole d'étude :

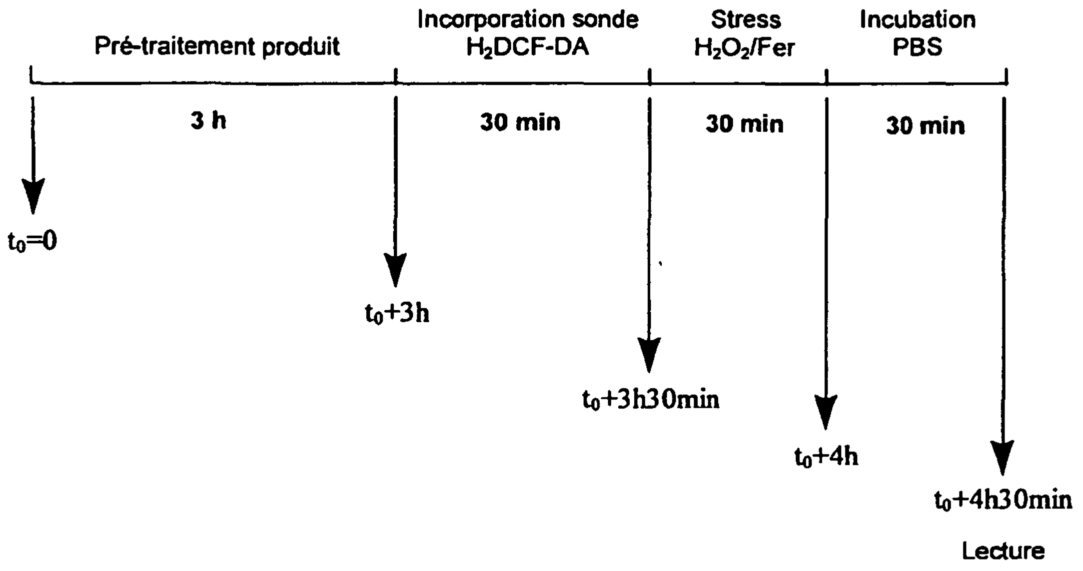


Calcul du % de protection contre la production d'EAO intracellulaires :

Le rapport ci-dessous permet de calculer pour chaque concentration de produit testé le % de protection contre la production d'EAO intracellulaires (puisque l'Intensité de Fluorescence ou IF exprime la libération intracellulaire d'EAO).

$$\frac{[\text{IF Oxydant }(H_2O_2/\text{Fer}) - \text{IF (Oxydant + Produit)}] \times 100}{\text{IF Oxydant }(H_2O_2/\text{Fer}) - \text{IF Contrôle}}$$

Les valeurs indiquées dans le tableau 3 sont les pourcentages d'inhibition de production d'EAO intracellulaire suite à un stress oxydatif exogène, par rapport aux cellules « témoin » (100%) et aux cellules « stimulé » (0%).

Les pourcentages moyens de protection, pour les 13 molécules testées à 4 concentrations sur la lignée L929 sont présentés dans le tableau 3 ci-dessous.

Tableau 3

| | Composés de formule (I) | | | | | Doses | | | |
|---|---|---|---|---|---|---|---|---|---|
| N° | n | R | Rn | R1 | Sel | 0,02 ng/ml | 0,2 ng/ml | 2 ng/ml | 20 ng/ml |
| 1 | 2 | H | H | H | HCl | 12 | 13 | 5 | 1 |
| 2 | 2 | H | H | F | HCl | 14 | 19 | 14 | -13 |
| 3 | 2 | H | H | F | HCl | 15 | 11 | 5 | 1 |
| 4 | 3 | H | H | H | HCl | 13 | 12 | 8 | -14 |
| 5 | 3 | H | H | F | HCl | 18 | 23 | 26 | 23 |
| 6 | 3 | H | H | F | HCl | 31 | 30 | 21 | 26 |
| 7 | 4 | H | H | H | HCl | 31 | 33 | 26 | 32 |
| 8 | 5 | H | H | H | HCl | 22 | 25 | 20 | 19 |
| 9 | 5 | H | H | F | HCl | 18 | 20 | 23 | 18 |
| 10 | 10 | H | H | F | HCl | 20 | 25 | 18 | 15 |
| 11 | 10 | H | H | H | HCl | 20 | 19 | 22 | 19 |

| Produit antiradicalaire de référence | Dose = 400 µg/ml |
|---|---|
| Vitamine E | 31 |

Conclusion :

In vitro, à l'échelle cellulaire, un stress exogène par le $H_2O_2$ / $Fe^{2+}$ -$Fe^{3+}$ est capable d'induire une production d'EAO intracellulaires détectés à l'aide de la sonde fluorescente.

> ➢ La vitamine E (molécule anti-radicalaire de référence) présente une protection moyenne de 31 % à 400 µg/ml.
> ➢ Les dérivés amino-acides gras insaturés selon l'invention à courte chaîne (C7 à C9), en particulier, les composés n° 8, 7 et 6, présentent des propriétés anti-radicalaires (activité > à 25 %).

5) Etude de l'effet anti-radicalaire. Analyse de la péroxydation lipidique

Par ailleurs, durant le métabolisme cellulaire normal, lors d'exposition occasionnelle de la peau à des agents stressants ou au cours de pathologies dermatologiques, des espèces oxygénées réactives encore appelées Radicaux Libres Oxygénés (RLO) sont générées (Y. M. W. Janssen *et al,* 1993). Ces RLO, décrits comme des métabolites très réactifs, jouent un rôle important dans bon nombre de processus tels que l'inflammation, le vieillissement et la promotion tumorale.

Les RLO sont considérés comme les « seconds messagers » dans la signalisation cellulaire du stress oxydatif et donc comme les médiateurs précoces de l'inflammation (A. Van Der Vliet and A. Bast, 1992).

Leur surproduction induit d'importants dommages au sein de la cellule. Certains constituants cellulaires sont alors les cibles majeures d'un tel stress oxydatif : les composants lipidiques de la membrane plasmique (lipoperoxydation), les protéines (dénaturation et dégradation) et le matériel génétique ou ADN (mutations) peuvent être altérés. Les

cellules sont capables de limiter ces dommages oxydatifs grâce à différents systèmes de défense antiradicalaire (antioxydants enzymatiques et non enzymatiques) (B. P. Yu, 1994 ; H. Steiling *et al,* 1999).

Cependant, dans certaines conditions, les RLO sont produits en quantité telle que l'activité antioxydante cellulaire est insuffisante ; ces RLO deviennent alors des facteurs inducteurs de pathologies inflammatoires et du vieillissement tissulaire (Y. Miyachi *et al*, 1986 ; M. Kress *et al,* 1995).

Afin de valoriser l'activité anti-radicalaire des différents dérivés selon l'invention, nous avons analysé leur pouvoir protecteur vis à vis de l'altération des membranes cellulaires induite par un stress oxydatif (chimique), en comparaison avec un antioxydant de référence, la vitamine E.

La membrane plasmique constitue la principale et la première cible des RLO et, étant riche en lipides, elle est le site d'une peroxydation accrue (A. W. Girotti, 1985). Les peroxydes générés au cours de cette oxydation lipidique sont aussi très réactifs et capables de dégrader le matériel protéique et génomique.

Pour évaluer l'altération membranaire, nous avons mesuré la peroxydation lipidique par un dosage *in vitro* des complexes entre les produits d'oxydation lipidique et l'acide thiobarbiturique. Ces complexes sont appelés TBARS (pour Thiobarbituric Acid Reactive Substances) et donnent le nom au test : Test des TBARS.

Afin de mimer un stress oxydatif chimique, nous avons traité la lignée de fibroblastes, L929, par un complexe composé de peroxyde d'hydrogène ($H_2O_2$) et de fer ($Fe^{2+}/Fe^{3+}$) reconstituant ainsi la réaction de Fenton, source de RLO et plus particulièrement de radical hydroxyle (OH°) (D.A Vessey *et al,* 1992) : $H_2O_2 + Fe^{2+} \rightarrow OH° + OH^- + Fe^{3+}$.

Les produits ont été évalués sur la lignée de fibroblastes murins L929. Les cellules sont prétraitées avec les différentes concentrations de produits pendant 16 heures et sont ensuite stimulées avec le complexe $H_2O_2$-$Fe^{2+}/Fe^{3+}$ (200$\mu$M-1mM) pendant 1 heure.

Solutions mères : 100 mg/ml, éthanol, 4°C.

Solutions finales : 0,02 ng/ml.

La peroxydation des lipides membranaires est analysée en mesurant les TBARS (Protocole Réf. PLN°2, selon Morlière *et al,* 1991).

*Principe du test :*

En milieu acide, à 95°C, se forment des complexes, notés TBARS pour Thio Barbituric Acid Reactive Substance, entre les produits d'oxydation lipidiques (malondialdéhyde ou MDA) et l'acide thiobarbiturique (TBA) qui peuvent être dosés en fluorescence par rapport à une gamme étalon avec le MDA. Le dosage des TBARS est alors exprimé en pmole/pg de protéines. Les protéines et TBARS sont dosés dans le milieu intracellulaire.

*Calcul du pourcentage de protection des membranes cellulaires :*

A partir du calcul des TBARS en pmole/$\mu$g protéines, nous avons calculé l'efficacité protectrice des différents produits contre l'oxydation des lipides membranaires.

$$\text{\% de protection : } \frac{[\text{TBARS Contrôle}] - [\text{TBARS (+produits)}]}{\text{TBARS Contrôle}} \times 100$$

Sept expériences indépendantes ont été réalisées. Durant ces expériences, divers composés ont été évalués (le test ne permet pas d'évaluer plus de 10 molécules en même temps). Les composés évalués plusieurs fois ont été choisis en fonction des résultats obtenus dans l'autre test mesurant également une activité anti-radicalaire (test de dosage des espèces actives de l'oxygène, EAO).

Le modèle utilisé dans cette expérience (réaction de Fenton) induit une peroxydation lipidique importante dans les fibroblastes L929. Cette décharge massive de radical hydroxyle OH- génère donc un stress oxydatif au niveau cellulaire et notamment au niveau des membranes. Cependant, dans ce type de réaction oxydative, les produits issus de la peroxydation lipidique sont internalisés dans les cellules et les TBARS sont alors dosés dans le milieu intracellulaire.

La vitamine E à 400$\mu$g/ml, diminue la peroxydation lipidique induite par le complexe $H_2O_2$-$Fe^{2+}/Fe^{3+}$, et protège très efficacement les membranes cellulaires.

Dans le tableau 4 suivant, sont présentés les résultats des 7 expériences.

Tableau 4

| Composés selon l'invention | | | | | | % de protection des lipides membranaires | |
|---|---|---|---|---|---|---|---|
| N° | n | R | $R_n$ | $R_1$ | Sel | Dose = 0,02 ng / ml | |
| | | | | | | Nbre Expériences | Moyenne |
| 1 | 2 | H | H | H | HCl | 3 | 13,41 |

(suite)

| Composés selon l'invention | | | | | | % de protection des lipides membranaires | |
|---|---|---|---|---|---|---|---|
| | | | | | | Dose = 0,02 ng / ml | |
| N° | n | R | $R_n$ | $R_1$ | Sel | Nbre Expériences | Moyenne |
| 2 | 2 | H | H | F | HCl | 3 | 7,63 |
| 3 | 2 | H | H | F | HCl | 3 | - 4,00 |
| 4 | 3 | H | H | H | HCl | 3 | 8,75 |
| 5 | 3 | H | H | F | HCl | 3 | 36,27 |
| 6 | 3 | H | H | F | HCl | 5 | 49,82 |
| 7 | 4 | H | H | H | HCl | 4 | 48,30 |
| 8 | 5 | H | H | H | HCl | 5 | 56,61 |
| 9 | 5 | H | H | F | HCl | 3 | 27,49 |
| 10 | 10 | H | H | F | HCl | 3 | 23,91 |
| 11 | 10 | H | H | H | HCl | 2 | -26,31 |

Conclusion :

Le module in vitro présenté dans cette étude reflète les conséquences dues à un stress oxydatif majeur sur la principale cible cellulaire qui est la membrane plasmique. Ainsi, le dosage de la peroxydation lipidique constitue un bon marqueur du stress oxydatif et permet l'évaluation de l'action antioxydante, vis-à-vis du radical hydroxyle, de principes actifs au niveau de la membrane cellulaire.

Dans nos conditions expérimentales, nous observons que les composés n° 8, 9, 10, 7, 5 et 6 présentent une activité antiradicalaire importante et protègent très efficacement les membranes des cellules.

Les composés n° 8, 7 et 6 présentent l'activité antiradicalaire et la protection des membranes les plus importantes.

6) Effet des dérivés d'amino-acides gras insaturés selon l'invention sur la synthèse de mélanine in vitro

Les mélanocytes sont des cellules d'aspect étoilé, présentes en minorité dans la couche basale de l'épiderme. Leur fonction principale est d'assurer la mélanogenèse, processus par lequel la mélanine est synthétisée dans des organites spécialisés, les mélanosomes, puis transportée et distribuée aux kératinocytes voisins via leurs prolongements dendritiques. Ce contact avec les kératinocytes permet la pigmentation cutanée, mécanisme de protection de l'épiderme contre les effets mutagènes des rayons ultraviolets. Chaque mélanocyte est en relation avec environ trente six kératinocytes, formant ainsi une « unité épidermique de mélanisation ».

La mélanogenèse consiste en une série de réactions enzymatiques et spontanées, dont le précurseur est la tyrosine. Trois enzymes principales participent à ce processus : la tyrosinase, et les tyrosinase-related protein 1 et 2 (TRP 1 et 2) (Jimbow et al., 2000). La tyrosinase catalyse la transformation de la tyrosine en dopaquinone. A partir de là, deux voies de synthèse sont alors possibles : l'eumélanogenèse et la phéomélanogenèse. La conversion de dopaquinone en eumélanine se fait par une série de réactions successives d'oxydations faisant intervenir TRP-1 et TRP-2. L'eumélanine correspond au pigment brun noir, à faible teneur en soufre, et assure le pouvoir photoprotecteur. Dans la phéomélanogenèse, des molécules à forte teneur en soufre s'incorporent à la dopaquinone pour donner la phéomélanine, de couleur jaune-orangé, présente dans les peaux des sujets roux.

Le stimulus physiologique de la synthèse de mélanine est le soleil, ce qui entraîne une augmentation du nombre des mélanocytes, une néosynthèse de mélanine, et des modifications morphologiques des mélanocytes, associant un accroissement de leur dendricité à une augmentation du transfert des mélanosomes aux kératinocytes. Au niveau moléculaire, l'exposition au soleil stimule la synthèse et la sécrétion d'alpha mélanocyte stimulating hormone. L'$\alpha$-MSH augmente la concentration intramélanocytaire en AMPc, activant le facteur de transcription, Mitf, qui stimule à son tour l'activité transcriptionnelle des gènes codant pour la tyrosinase, TRP-1 et TRP-2 .

Certaines molécules exogènes sont également connues pour réguler négativement la mélanogenèse. L'hydroquinone inhibe la synthèse de mélanine en se présentant comme substrat de la tyrosinase afin de détourner son activité (Curto et al., 1999). La vitamine C inhibe la tyrosinase mais se comporte également comme un réducteur puissant

en empêchant la coloration de la mélanine par oxydation.

6.1) L'effet modulateur des dérivés d'amino acides gras insaturés, sur la mélanogénèse a été analysé. Pour cela une mesure de la synthèse de mélanine par dosage colorimétrique est effectuée sur une lignée cellulaire de mélanomes murins : la lignée B16-F10.

L'effet des produits est investigué en situation basale et après stimulation de la mélanogénèse par α MSH pour déterminer le pouvoir dépigmentant.

Dosage de la mélanine :

Le taux de mélanine extracellulaire et intracellulaire est alors mesuré par spectrophotométrie à une longueur d'onde de 405 nm selon le protocole (Meun, Y. J. et al.). La quantité de pigment est déterminée grâce à une gamme étalon de mélanine et l'analyse sur le logiciel Microwin (Berthold Biotechnologies). Un dosage de protéines totales est effectué pour les échantillons de mélanine intracellulaire par la méthode BCA-Copper à 540nm. La gamme étalon est réalisée avec une protéine standard, la BSA (Serum Albumine Bovine).

6.2) RESULTATS :

En situation basale l'alpha MSH à 1 μM augmente de plus de 100% la production de mélanine par rapport aux cellules témoins.

Cette augmentation de mélanine est contrée par les agents dépigmentants, tels l'hydroquinone à 1 μg/ml ou la vitamine C à 40 μg/ml, qui inhibent près de 60% la mélanogénèse.

● Molécule dépigmentante:

A 30 μg/ml, la molécule n° 11 inhibe faiblement la quantité de mélanine intra et extracellulaire respectivement à hauteur environ de 45 % et de 30 %. Toutefois, la molécule n° 11 reste sans effet sur la synthèse de la mélanine à 10 μg/ml.

● Molécules pigmentantes :

A 3, 10 et 30 μg/ml, la molécule n° 6 augmente la quantité de mélanine intracellulaire à hauteur d'environ 100 %. La molécule n° 5 semble également avoir un effet pigmentant, mais il est plus faible que celui de la molécule n° 6.

7) Etude de l'activité anti- collagénase

Pour mesurer l'activité anti-collagénase, on peut notamment se référer à la demande de brevet français n° 2 829 491 publiée le 14 mars 2003.

7.1. Sur coupes congelées de peau humaine (exemple comparatif)

7.1.1. PROTOCOLE

Cette étude est réalisée sur différentes solutions à la concentration de 1 % et 2% de principes actifs en comparaison avec l'excipient seul, les témoins tampon et collagénase. Les principes actifs utilisés sont le DHA, le 2-diméthylamino éthyl ester de l'acide hydroxy-10-décène-2(trans)oïque (ML40) et le glycérol ester de l'acide hydroxy-10-décène-2(trans)oïque (GM).

Des coupes congelées de 5 μm, provenant d'une plastie mammaire d'une femme de 54 ans, sont placées sur des lames histologiques (4 coupes par lame). Chaque solution est testée sur une lame.

Les coupes sont recouvertes des solutions à tester puis mises à incuber pendant 2 heures à 37°C en chambre humide. Les solutions sont éliminées par rinçages répétitifs et les coupes sont colorées au picrosirius. Un examen microscopique est réalisé.

7.1.2. RESULTATS

Les produits de la présente invention ont une activité supérieure à l'activité précédemment connue DHA.

7.2. Sur explants de peau humaine maintenue en survie.

7.2.1. PROTOCOLE

L'étude est faite sur un produit à 5% de GM en comparaison avec de l'excipient

(hydrocérine), un contrôle positif et un témoin en présence de la collagénase à 100 U/ml. L'hydrocérine est utilisé comme excipient pour la préparation du produit à appliquer.

Cette étude a été réalisée deux fois. Dans la première étude, il a été constaté que l'action de la collagénase à J+2 reste très limitée et non significative. Dans la deuxième étude, le temps d'application est prolongé et le prélèvement des explants est effectué à J+2 et à J+4.

7.2.1.1. Préparation des explants.

Des explants de peau humaine préparés et repartis en 16 lots de trois explants chacun sont mis en survie selon le tableau 5 :

Tableau 5

| | J+2 | J+4 |
|---|---|---|
| Témoin | 3 explants | 3 explants |
| Excipient | 3 explants | 3 explants |
| Produit & 5% GM | 3 explants | 3 explants |
| Contrôle positif | 3 explants | 3 explants |
| Témoin + collagénase | 3 explants | 3 explants |
| Excipient + collagénase | 3 explants | 3 explants |
| Produit 2 5% GM + collagénase | 3 explants | 3 explants |
| Contrôle positif + collagénase | 3 explants | 3 explants |

7.2.1.2. Application du produit à 5% de GM et de produits issus de la présente invention

Le produit est appliqué à J0 et à J+2 à raison de 20 mg par explant et la collagénase est incorporée dans les milieux de culture des 24 derniers lots.

7.2.1.3. Histologie

Trois explants de chaque lot sont prélevés à J+2 et J+5 J+4 fixés au Bouin ordinaire et traités en histologie. L'étude histologique comprend :

- imprégnation en paraffine,
- coupes, coloration au rouge sirius F3B
- Mesures colorimétriques du collagène par analyse d'images
- Rapport avec photos.

7.2.2. RESULTATS

Les prélèvements réalisés à J+2 ne montrent aucune activité significative de la collagénase quelque soit le lot examiné. Pour cette raison, la survie, le contact et l'application sont prolongés jusqu'à J+4. L'action de la collagénase est notée de 2 manières : intensité de la coloration du réseau de collagène et épaisseur de la structure dermique. Avec cette étude, sont corrélées à la pénétration du principe actif et à son activité inhibitrice vis-à-vis de la collagénase. Les résultats obtenus sont les suivants :

- pour les témoins sans collagénase le derme présente une structure normale, avec des faisceaux de collagène réguliers dans tous les compartiments,
- pour les témoins avec collagénase, les faisceaux de collagène sont très fortement dégrades et l'épaisseur du derme a diminue de moitié,
- pour les explants avec excipient et collagénase, les faisceaux de collagène sont fortement dégradés, l'altération étant inférieure à celle observée sur les témoins avec collagénase, et l'épaisseur du derme a diminué de presque

moitié.

Par comparaison avec le produit GM, les produits issus de l'invention montrent une activité anti-collagénase supérieure et plus rapide que les esters du DHA (GM).

Bibliographie :

- Janssen Y, M, W, et al (1993) Lab, Invest, 69:261-74
- Van Der Vliet A, and Bast A, (1992) Chem, Biol, Interactions 85 : 95-116
- Yu B, P, (1994) Physiol, Rev, 74: 139-62
- Steiling H, et al (1999) Exp. Cell, Res, 247 :484-94
- Miyachi Y, et al (1986) J, Clin, Lab, Immunol, 19: 11-4
- Kress M, et al (1995) Pain 62 : 87-94
- Girotti A, W, (1985) J, Free Radic, Biol, Med, 1 : 87-95
- Vessey D, A et al (1992) J, Invest, Dermatol, 99 : 859-63
- Morlière P, et al, (1991) Biochim, Biophys, Acta, 1084 : 261-268
- Emonet E et al (1997) J. Photochem. Photobiol 40 : 84-90
- Meun, Y. J. et al. (2004) Biol. Pharm. Bull. June, 27 (n° 6) : 806 - 809
- Curto EV., Kwong C., Hermersdörfer H., Glatt H., Santis C., Virador V., Hearing VJ., Dooley TP. Inhibitors of mammalian melanocyte tyrosinase: in vitro comparisons of alkyl esters of gentisic acid with others putative inhibitors (1999) Biochemical Pharmacology 57: 663-672
- Jimbow K., Hua C., Gomez P., Hirosaki K., Shinoda K., Salopek T., Matsusaka H., Jin H., Yamashita T. Intracellular vesicular trafficking of tyrosinase gene family protein in eu- and pheomelanosome biogenesis (2000) Pigment cell res. 13: 110-117


**Revendications**

1. A titre de médicaments, dérivés d'amino-acides gras insaturés répondant à la formule générale (I) :

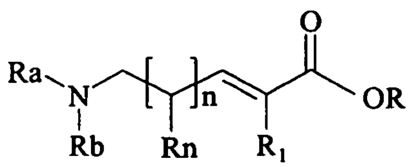

(I)

sous forme d'isomères Z ou E, ou d'un mélange d'isomères Z et d'isomères E, en toutes quantités, ainsi que leurs sels d'addition d'acides pharmaceutiquement acceptables, dans laquelle :

● Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;

● $R_1$ représente un atome d'hydrogène, de fluor, de chlore, ou de brome, ou bien un groupement $-CF_3$ ou $-CHF_2$ ou bien un groupement alkyle, alkényle comportant au moins une double liaison ou alkynyle comportant au moins une triple liaison, linéaire ou ramifié, comprenant 1 à 6 atomes de carbone, et étant éventuellement substitué par un atome d'halogène, en particulier de fluor ;

● R représente un atome d'hydrogène ou un groupement R' représentant un groupement alkyle, linéaire ou ramifié, comprenant 1 à 6 atomes de carbone, ou cycloalkyle comprenant 3 à 6 atomes de carbone, et étant éventuellement substitué par un atome d'halogène, en particulier de fluor ;

● Ra et Rb représentent indépendamment l'un de l'autre un atome d'hydrogène ou un groupement alkyle ou acyle, linéaire ou ramifié, comprenant 1 à 6 atomes de carbone, Ra et Rb pouvant former ensemble un cycle hydrocarboné comportant de 4 à 6 atomes de carbone ; et

• n est un entier compris entre 2 et 14.

2. Dérivés d'amino-acides gras insaturés de formule générale (I) selon la revendication 1, **caractérisés en ce que**

Ra et Rb représentent un atome d'hydrogène.

3. Dérivés d'amino-acides gras insaturés de formule générale (I) selon l'une quelconque des revendications 1 et 2, **caractérisés en ce que** R représente un atome d'hydrogène.

4. Dérivés d'amino-acides gras insaturés de formule générale (I) selon l'une quelconque des revendications 1 à 3, **caractérisés en ce que** Rn représentent un atome d'hydrogène.

5. Dérivés d'amino-acides gras insaturés de formule générale (I) selon l'une quelconque des revendications 2 à 4, **caractérisé en ce que** $R_1$ représente un atome d'hydrogène ou de fluor.

6. Dérivés d'amino-acides gras insaturés de formule générale (I) selon l'une quelconque des revendications 2 à 5, **caractérisé en ce que** n est compris entre 2 et 10, notamment entre 3 et 5, et en particulier vaut 3.

7. Dérivés d'amino-acides gras insaturés de formule générale (I) selon l'une quelconque des revendications 2 à 5, **caractérisé en ce que** n est compris entre 9 et 14.

8. Dérivés d'amino-acides gras insaturés de formule générale (I) selon l'une quelconque des revendications 1 à 7, **caractérisés en ce qu'**ils sont choisis parmi :

   - le chlorhydrate de l'acide (*E*)-6-amino-hex-2-énoïque,
   - le chlorhydrate de l'acide (*E*)-7-amino-hept-2-énoïque,
   - le chlorhydrate de l'acide (*E*)-8-amino-oct-2-énoïque,
   - le chlorhydrate de l'acide (*E*)-9-amino-non-2-énoïque,
   - le chlorhydrate de l'acide (*E*)-10-amino-déc-2-énoïque,
   - le chlorhydrate de l'acide (*E*)-14-amino-tétradéc-2-énoïque,
   - le chlorhydrate de l'acide 6-amino-2-fluoro-hex-2-énoïque sous forme d'un mélange d'isomères Z et E,
   - le chlorhydrate de l'acide (*Z*)-6-amino-2-fluoro-hex-2-énoïque,
   - le chlorhydrate de l'acide (*E*)-6-amino-2-fluoro-hex-2-énoïque,
   - le chlorhydrate de l'acide 7-amino-2-fluoro-hept-2-énoïque sous forme d'un mélange d'isomères Z et E,
   - le chlorhydrate de l'acide (*Z*)-7-amino-2-fluoro-hept-2-énoïque,
   - le chlorhydrate de l'acide 9-amino-2-fluoro-non-2-énoïque,
   - le chlorhydrate de l'acide 10-amino-2-fluoro-déc-2-énoïque, et
   - le chlorhydrate de l'acide 14-amino-2-fluoro-tétradéc-2-énoïque.

9. A titre de composés chimiques nouveaux, dérivés d'amino-acides gras insaturés répondant à la formule générale (I) telle que définie dans l'une quelconque des revendications 1 à 8, à l'exclusion des composés suivants : l'acide 6-amino-hex-2-ènoïque, son chlorhydrate et son trifluoroacétate, l'acide 11-amino-2-undecenoïque, le trifluoroacétate d'acide 8-amino-oct-2-ènoïque, l'acide 8-(diméthylamino)-oct-2-ènoïque, l'acide 12-(diméthylamino)-dodéc-2-ènoïque, le 6-(isopropylamino)-2-méthyl-hex-2-énoate d'éthyle, le 6-(*tert*-butylamino)-2-méthyl-hex-2-énoate d'éthyle, le 6-amino-2-méthyl-hex-2-énoate de méthyle, le 7-amino-hept-2-énoate de méthyle, le 7-amino-2-méthyl-hept-2-énoate de méthyle et le 7-amino-4-méthyl-hept-2-énoate de méthyle.

10. Compositions dermatologiques comprenant à titre de principe actif au moins un dérivé d'amino-acide gras insaturé selon l'une quelconque des revendications 1 à 8, en association avec un excipient dermatologiquement acceptable.

11. Utilisation d'un dérivé d'amino-acide gras insaturé selon l'une quelconque des revendications 1 à 8, pour la préparation d'une composition dermocosmétologique antiradicalaire, anti-inflammatoire, anti-prurigineuse, anti-collagénase, et/ou destinée au traitement du vieillissement cutané, et/ou destinée au traitement des troubles de la kératinisation et de la pigmentation, et/ou destinée à améliorer la cicatrisation.

12. Utilisation selon la revendication 11, **caractérisée en ce que** ladite composition est destinée au traitement du psoriasis, du prurit et/ou de la dermite atopique ; ou à repigmenter les cheveux ou la peau, notamment les taches de vieillesse blanches ; ou à provoquer un éclaircissement de la peau ou à traiter les taches de vieillesse brunes.

13. Procédé de préparation d'un dérivé d'aminoacide gras insaturé de formule générale (I) suivante :

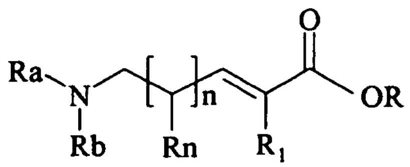

(I)

dans laquelle:

- Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;
- $R_1$ représente un atome d'hydrogène, de fluor, de chlore, ou de brome, ou bien un groupement $-CF_3$ ou $-CHF_2$ ou bien un groupement alkyle, alkényle comportant au moins une double liaison ou alkynyle comportant au moins une triple liaison, linéaire ou ramifié, comprenant 1 à 10 atomes de carbone, et étant éventuellement substitué par un atome d'halogène, en particulier de fluor ;
- R représente un atome d'hydrogène ou un groupement R' représentant un groupement alkyle, linéaire ou ramifié, comprenant 1 à 6 atomes de carbone, ou cycloalkyle comprenant 1 à 6 atomes de carbone, et étant éventuellement substitué par un atome d'halogène, en particulier de fluor ;
- Ra et Rb représentent indépendamment l'un de l'autre un atome d'hydrogène ou un groupement alkyle ou acyle, linéaire ou ramifié, comprenant 1 à 6 atomes de carbone, Ra et Rb pouvant former ensemble un cycle hydrocarboné comportant de 4 à 6 atomes de carbone ; et
- n est un entier compris entre 2 et 14 ;

ledit procédé de préparation comprenant :

- la mise en oeuvre de la réaction de Wittig-Horner par la réaction d'un phosphonate de formule générale (II) suivante :

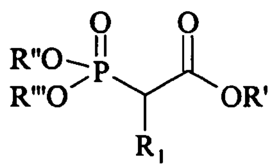

(II)

dans laquelle :

- $R_1$ est tel que défini ci-dessus,
- R' représente un groupement alkyle, linéaire ou ramifié, comprenant de 1 à 6 atomes de carbone, et est de préférence un groupement éthyle, et
- R" et R"' représentent, indépendamment l'un de l'autre, un groupe alkyle, linéaire ou ramifié, comprenant de 1 à 6 atomes de carbone, et de préférence un groupe éthyle ou méthyle, lesdits groupes R" et R"' pouvant former un cycle hydrocarboné comprenant de 2 à 4 atomes de carbone,

ou, lorsque $R_1$ = H, la mise en oeuvre de la réaction de Doebner-Knoevenagel par la réaction d'un dérivé d'acide malonique de formule $R'OOC-CH_2-COOR'$, R' étant tel que défini ci-dessus,
sur un composé de formule générale (III) suivante :

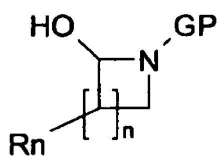

(III)

dans laquelle :

• n est tel que défini ci-dessus, et
• GP représente un groupe protecteur, N-GP représentant avantageusement un groupe de type carbamate et de préférence un carbamate de *tert*-butyle ou de benzyle, afin d'obtenir un composé de formule générale (IV) suivante :

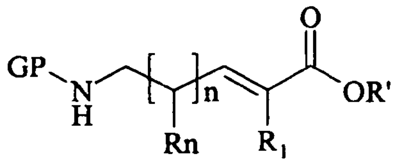

(IV)

pour lequel GP, R', $R_1$, Rn et n sont tels que définis ci-dessus,

- une éventuelle réaction de saponification du composé de formule générale (IV) susmentionnée, pour obtenir un composé de formule générale (V) suivante :

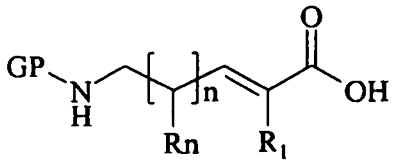

(V)

pour lequel GP, $R_1$, Rn et n sont tels que définis ci-dessus,

- une réaction de déprotection de l'azote du composé de formule (IV) ou (V) susmentionnée, pour obtenir un composé de formule (I) dans laquelle Ra et Rb représentent un hydrogène,
- et une éventuelle réaction de *N*-alkylation ou *N*-acylation du composé de formule (I) précédent dans laquelle Ra et Rb représentent un hydrogène, pour obtenir un composé de formule (I) dans laquelle au moins l'un des groupes Ra et Rb ne représente pas un hydrogène.

**14.** Procédé selon la revendication 13, **caractérisé en ce que** le composé de formule générale (III) :

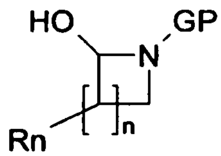

(III)

est synthétisé à partir d'un composé de formule générale (VI) :

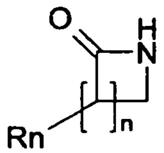

(VI)

dans laquelle n et Rn sont tels que définis dans la revendication 13
selon les étapes suivantes :

- protection de l'azote de la fonction amide du composé de formule (VI) par un groupement GP pour obtenir un composé de formule générale (VII) suivante :

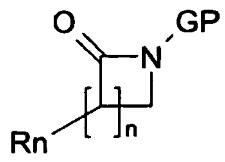

(VII)

dans laquelle n, Rn et GP sont tels que définis dans la revendication 13

- puis réduction de la fonction carbonyle du composé de formule (VII) tel que défini ci-dessus, cette étape étant notamment réalisée à l'aide d'hydrures d'aluminium tel que l'hydrure de diisobutyle aluminium à basse température.

**15.** A titre d'intermédiaires de synthèse, les composés de formule générale (VIII) :

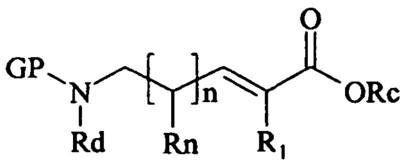

(VIII)

pour laquelle -

- Rc représente un hydrogène ou un groupement alkyle, linéaire ou ramifié, comportant de 1 à 6 atomes de carbone ;
- $R_1$ représente un atome d'hydrogène, de fluor, de chlore, ou de brome, ou bien un groupement $-CF_3$ ou $-CHF_2$ ou bien un groupement alkyle, alkényle comportant au moins une double liaison ou alkynyle, comportant au moins une triple liaison linéaire ou ramifié, comprenant 1 à 6 atomes de carbone, et étant éventuellement substitué par un ou plusieurs atomes d'halogène, en particulier de fluor ;
- Rn représentent indépendamment les uns des autres un atome d'hydrogène ou un groupement alkyle, linéaire ou ramifié, comprenant 1 à 6 atomes de carbone, et étant éventuellement substitué par un ou plusieurs atomes d'halogène, en particulier de fluor ;
- n est un entier compris entre 2 et 14 ;
- GP représente un groupe protecteur, N-GP représentant avantageusement un groupe de type carbamate et de préférence un carbamate de *tert*-butyle ou de benzyle,
- Rd représente un hydrogène,

à l'exclusion des composés suivants : le (*E*)-6-(*N*,*N*-di-*tert*-butoxy-carbonylamino)-hex-2-énoate de *tert*-butyle, le (*E*)-6-(*N*-*tert*-butoxy-carbonylamino)-hex-2-énoate de *tert*-butyle, le (*E*)-6-(*N*-*tert*-butoxy-carbonylamino)-hex-2-énoate d'éthyle, le (*E*)-7-(*N*-*tert*-butoxy-carbonylamino)-hept-2-énoate de méthyle et l'acide (*E*)-7-(*N*-*tert*-butoxy-carbonylamino)-hept-2-énoïque.

**16.** Composé selon la revendication 15, **caractérisé en ce que** Rn représentent un hydrogène.

**17.** Composé selon la revendication 16, **caractérisé en ce que** $R_1$ représente un hydrogène ou un fluor.

## Claims

**1.** Unsaturated fatty amino-acid derivatives, as drugs, fitting the general formula (I):

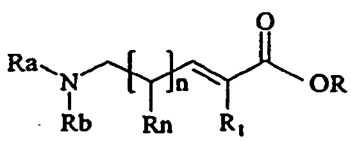

(I)

in the form of Z or E isomers, or a mixture of Z and E isomers, in all quantities,
as well as their pharmaceutically acceptable acid addition salts, wherein:

- Rn represent independently of each other a hydrogen atom or a linear or branched alkyl group comprising 1-6 carbon atoms, and being optionally substituted with one or more halogen atoms, in particular fluorine;
- $R_1$ represents a hydrogen, fluorine, chlorine or bromine atom, or else a $-CF_3$ or $-CHF_2$ group or else a linear or branched alkyl group, alkenyl group comprising at least one double bond, or alkynyl group comprising at least one triple bond, comprising 1-6 carbon atoms, and being optionally substituted with a halogen atom, in particular fluorine;
- R represents a hydrogen atom or a group R' representing a linear or branched alkyl group comprising 1-6 carbon atoms, or a cycloalkyl group comprising 3-6 carbon atoms, and being optionally substituted with a halogen atom, in particular fluorine;
- Ra and Rb represent independently of each other a hydrogen atom or a linear or branched alkyl or acyl group comprising 1-6 carbon atoms, Ra and Rb may form together a hydrocarbon cycle including 4-6 carbon atoms; and
- n is an integer comprised between 2 and 14.

**2.** Unsaturated fatty amino-acid derivatives of general formula (I) according to claim 1, **characterized in that** Ra and Rb represent a hydrogen atom.

**3.** Unsaturated fatty amino-acid derivatives of general formula (I) according any of claims 1 and 2, **characterized in that** R represents a hydrogen atom.

**4.** Unsaturated fatty amino-acid derivatives of general formula (I) according to any of claims 1 to 3, **characterized in that** Rn represents a hydrogen atom.

**5.** Unsaturated fatty amino-acid derivatives of general formula (I) according to any of claims 2 to 4, **characterized in that** $R_1$ represents a hydrogen or fluorine atom.

**6.** Unsaturated fatty amino-acid derivatives of general formula (I) according to any of claims 2 to 5, **characterized in that** n is comprised between 2 and 10, in particular between 3 and 5, and in particular is 3.

**7.** Unsaturated fatty amino-acid derivatives of general formula (I) according to any of claims 2 to 5, **characterized in that** n is comprised between 9 and 14.

**8.** Unsaturated fatty amino-acid derivatives of general formula (I) according to any of claims 1 to 7, **characterized in that** they are selected from:

- (E)-6-amino-hex-2-enoic acid hydrochloride,
- (E)-7-amino-hept-2-enoic acid hydrochloride,
- (E)-8-amino-oct-2-enoic acid hydrochloride,
- (E)-9-amino-non-2-enoic acid hydrochloride,
- (E)-10-amino-dec-2-enoic acid hydrochloride,
- (E)-14-amino-tetradec-2-enoic acid hydrochloride,
- 6-amino-2-fluoro-hex-2-enoic acid hydrochloride as a mixture of isomers Z and E,
- (Z)-6-amino-2-fluoro-hex-2-enoic acid hydrochloride,
- (E)-6-amino-2-fluoro-hex-2-enoic acid hydrochloride,
- 7-amino-2-fluoro-hept-2-enoic acid hydrochloride as a mixture of isomers Z and E,
- (Z)-7-amino-2-fluoro-hept-2-enoic acid hydrochloride,
- 9-amino-2-fluoro-non-2-enoic acid hydrochloride,
- 10-amino-2-fluoro-dec-2-enoic acid hydrochloride, and
- 14-amino-2-fluoro-tetradec-2-enoic acid hydrochloride

**9.** Unsaturated fatty amino-acid derivative, as novel chemical compounds, fitting general formula (I) as defined according to any of claims 1 to 8, the following compounds being excluded: 6-amino-hex-2-enoic acid, its hydrochloride and its trifluoroacetate, 11-amino-2-undecenoic acid, 8-amino-oct-2-enoic acid trifluoro-acetate, 8-(dimethylamino)-oct-2-enoic acid, 12-(dimethyl-amino)-dodec-2-enoic acid, ethyl 6-(isopropylamino)-2-methyl-hex-2-enoate, ethyl 6-(tert-butylamino)-2-methyl-hex-2-enoate, methyl 6-amino-2-methyl-hex-2-enoate, methyl 7-amino-hept-2-enoate, methyl 7-amino-2-methyl-hept-2-enoate and methyl 7-amino-4-methyl-hept-2-enoate.

**10.** Dermatological compositions comprising as an active ingredient, at least one unsaturated fatty amino-acid derivative according to any of claims 1 to 8, in association with a dermatologically acceptable excipient.

**11.** Use of an unsaturated fatty amino-acid derivative according to any of claims 1 to 8, for preparing an anti-radical, anti-inflammatory, anti-pruriginous, anti-collagenase dermatocosmetological composition and/or intended for treating skin ageing and/or intended for treating keratinisation and pigmentation disorders, and/or intended to improve healing.

**12.** Use according to claim 11, **characterized in that** said composition is intended for treating psoriasis, pruritus, and/or atopic dermatitis; or for repigmenting hair or skin, notably white age spots; or for causing lightening of the skin or for treating brown age spots.

**13.** Process for preparing an unsaturated fatty amino acid derivative of the following general formula (I):

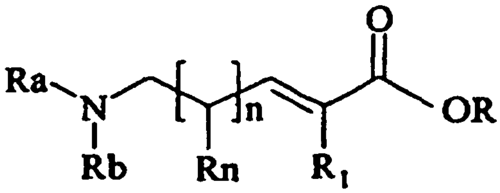

(I)

wherein:

- Rn represent independently of each other a hydrogen atom or a linear or branched alkyl group comprising 1-6 carbon atoms, and being optionally substituted with one or more halogen atoms, in particular fluorine;
- R1 represents a hydrogen, fluorine, chlorine or bromine atom, or else a -CF3 or -CHF2 group or else a linear or branched alkyl group, alkenyl group comprising at least one double bond or alkynyl group comprising at least one triple bond, comprising 1-10 carbon atoms, and being optionally substituted with a halogen atom, in particular fluorine;
- R represents a hydrogen atom or a group R' representing a linear or branched alkyl group comprising 1-6 carbon atoms, or a cycloalkyl group comprising 1-6 carbon atoms, and being optionally substituted with a halogen atom, in particular fluorine;
- Ra and Rb represent independently of each other a hydrogen atom or a linear or branched alkyl or acyl group comprising 1-6 carbon atoms, Ra and Rb may form together a hydrocarbon cycle including 4-6 carbon atoms; and
- n is an integer comprised between 2 and 14;

said preparation method comprising:

- the application of the Wittig-Horner reaction by reacting a phosphonate of the following general formula (II) :

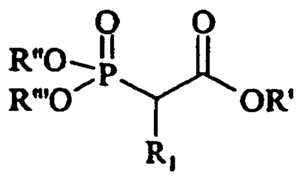

(II)

wherein:

- R1 is as defined above,
- R' represents a linear or branched alkyl group, comprising 1-6 carbon atoms, and is preferably an ethyl group, and
- R" and R"' represent independently of each other, a linear or branched alkyl group, comprising 1-6 carbon atoms, and preferably an ethyl or methyl group, said groups R" and R"' may form an hydrocarbon cycle comprising 2-4 carbon atoms,

or, when $R_1$ = H, the application of the Doebner-Knoevenagel reaction by reacting a malonic acid derivative of formula R'-OOC-$CH_2$-COOR', R' being as defined above,
on a compound of the following formula (III):

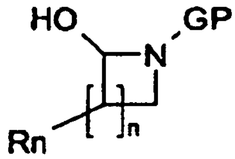

(III)

wherein:

- n is as defined above, and
- GP represents a protective group, N-GP advantageously representing a carbamate type group and preferably a ter-butyl or benzyl carbamate,

in order to obtain a compound of the following general formula (IV):

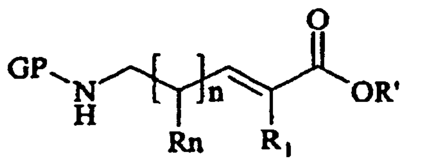

(IV)

for which GP, R', $R_1$, Rn and n are as defined above,

- an optional saponification reaction of the compound of the aforementioned general formula (IV), in order to obtain a compound of the following general formula (V):

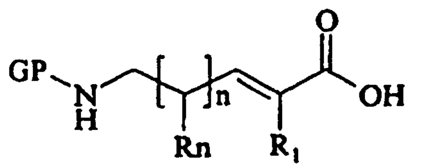

(V)

for which GP, $R_1$, Rn and n are as defined above,

- a reaction for deprotecting the nitrogen of the compound of the aforementioned formula (IV) or (V), in order to obtain a compound of formula (I) wherein Ra and Rb represent a hydrogen,
- and an optional N-alkylation or N-acylation reaction of the preceding compound of formula (I) wherein Ra and Rb represent a hydrogen, in order to obtain a compound of formula (I) wherein at least one of the Ra and Rb groups does not represent a hydrogen.

**14.** Process according to claim 13, **characterized in that** the compound of general formula (III):

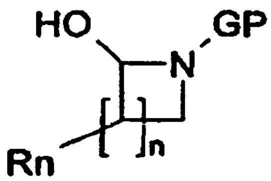

(III)

is synthesized from a compound of general formula (VI):

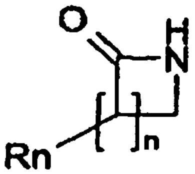

(VI)

wherein n and Rn are as defined in claim 13
according to the following steps:

- protection of the nitrogen of the amide function of the compound of formula (VI) with a GP group in order to obtain a compound of the following general formula (VII) :

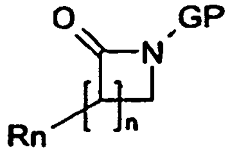

(VII)

wherein n, Rn and GP are as defined in claim 13,

- then reduction of the carbonyl function of the compound of formula (VII) as defined above, this step in particular being performed using aluminum hydrides such as aluminum diisobutyl hydride at low temperature.

**15.** Compounds of general formula (VIII), as synthesis intermediates :

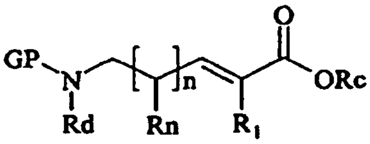

(VIII)

for which:

- Rc represents a hydrogen or a linear or branched alkyl group comprising 1-6 carbon atoms,
- $R_1$ represents a hydrogen, fluorine, chlorine or bromine atom, or else a $-CF_3$ or $-CHF_2$ group or else a linear or branched alkyl group, alkenyl group comprising at least one double bond, or alkynyl group comprising at least one triple bond, comprising 1-6 carbon atoms, and being optionally substituted with one or more halogen atoms, in particular fluorine;
- Rn represent independently of each other a hydrogen atom or a linear or branched alkyl group comprising 1-6 carbon atoms, and being optionally substituted with one or more halogen atoms, in particular fluorine;
- n is an integer comprised between 2 and 14;
- GP represents a protective group, N-GP advantageously representing a carbamate type group and preferably a tert-butyl or benzyl carbamate;
- Rd represents a hydrogen,

the following compounds being excluded: tert-butyl(E)-6-(N,N-di-tert-butoxy-carbonylamino)-hex-2-enoate, tert-butyl (E)-6-N-tert-butoxy-carbonylamino)-hex-2-enoate, ethyl(E)-6-(N-tert-butoxy-carbonylamino)-hex-2-enoate, methyl (E)-7-(N-tert-butoxy-carbonylamino)-hept-2-enoate, and (E)-7-(N-tert-butoxy-carbonylamino)-hept-2-enoic acid.

**16.** The compound according to claim 15, **characterized in that** Rn represent a hydrogen.

**17.** The compound according to claim 16, **characterized in that** $R_1$ represents a hydrogen or a fluorine.

## Patentansprüche

1. Derivate von ungesättigten Aminofettsäuren gemäß der allgemeinen Formel (I) als Medikamente:

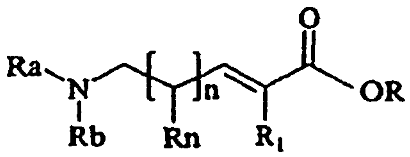

(I)

in Form von Z- oder E-Isomeren oder einer Mischung von Z-Isomeren und E-Isomeren in jeglichen Mengen, sowie deren pharmazeutisch akzeptablen Additionssalze, wobei:

• Rn jeweils unabhängig voneinander ein Wasserstoffatom oder eine lineare oder verzweigte Alkylgruppe mit 1 bis 6 Kohlenstoffatomen repräsentiert, ggf. substituiert durch ein oder mehrere Halogenatome, insbesondere Fluoratome;
• $R_1$ ein Wasserstoff-, Fluor-, Chlor- oder Bromatom repräsentiert, oder auch eine $CF_3$- oder $CHF_2$-Gruppe oder auch eine lineare oder verzweigte Alkylgruppe, Alkenylgruppe mit mindestens einer Doppelbindung oder Alkinylgruppe mit mindestens einer Dreifachbindung, umfassend 1 bis 6 Kohlenstoffatome, ggf. substituiert durch ein Halogenatom, insbesondere ein Fluoratom;
• R ein Wasserstoffatom oder eine Gruppe R' repräsentiert, welchletztere eine lineare oder verzweigte Alkylgruppe mit 1 bis 6 Kohlenstoffatomen oder eine Cycloalkylgruppe mit 3 bis 6 Kohlenstoffatomen repräsentiert, ggf. substituiert durch ein Halogenatom, insbesondere ein Fluoratom;
• Ra und Rb unabhängig voneinander ein Wasserstoffatom oder eine lineare oder verzweigte Alkyl- oder Acylgruppe mit 1 bis 6 Kohlenstoffatomen repräsentieren, wobei Ra und Rb gemeinsam einen ringförmigen Kohlenwasserstoff mit 4 bis 6 Kohlenstoffatomen bilden können; und
• n eine ganze Zahl zwischen 2 und 14 ist.

2. Derivate von ungesättigten Aminofettsäuren der allgemeinen Formel (I) nach Anspruch 1, **dadurch gekennzeichnet, dass** Ra und Rb ein Wasserstoffatom repräsentieren.

3. Derivate von ungesättigten Aminofettsäuren der allgemeinen Formel (I) nach einem der Ansprüche 1 und 2, **dadurch gekennzeichnet, dass** R ein Wasserstoffatom repräsentiert.

4. Derivate von ungesättigten Aminofettsäuren der allgemeinen Formel (I) nach einem der Ansprüche 1 bis 3, **dadurch gekennzeichnet, dass** Rn ein Wasserstoffatom repräsentieren.

5. Derivate von ungesättigten Aminofettsäuren der allgemeinen Formel (I) nach einem der Ansprüche 2 bis 4, **dadurch gekennzeichnet, dass** $R_1$ ein Wasserstoffatom oder ein Fluoratom repräsentiert.

6. Derivate von ungesättigten Aminofettsäuren der allgemeinen Formel (I) nach einem der Ansprüche 2 bis 5, **dadurch gekennzeichnet, dass** n zwischen 2 und 10 liegt, insbesondere zwischen 3 und 5, und insbesondere den Wert 3 hat.

7. Derivate von ungesättigten Aminofettsäuren der allgemeinen Formel (I) nach einem der Ansprüche 2 bis 5, **dadurch gekennzeichnet, dass** n zwischen 9 und 14 liegt.

8. Derivate von ungesättigten Aminofettsäuren der allgemeinen Formel (I) nach einem der Ansprüche 1 bis 7, **dadurch gekennzeichnet, dass** sie ausgewählt sind aus:

- dem Hydrochlorid der (*E*)-6-Amino-hex-2-ensäure,
- dem Hydrochlorid der (*E*)-7-Amino-hept-2-ensäure,
- dem Hydrochlorid der (*E*)-8-Amino-oct-2-ensäure,
- dem Hydrochlorid der (*E*)-9-Amino-non-2-ensäure,
- dem Hydrochlorid der (*E*)-10-Amino-dec-2-ensäure,
- dem Hydrochlorid der (*E*)-14-Amino-tetradec-2-ensäure,
- dem Hydrochlorid der 6-Amino-2-fluor-hex-2-ensäure in Form einer Mischung der Z- und E-Isomere,

- dem Hydrochlorid der (*Z*)-6-Amino-2-fluor-hex-2-ensäure,
- dem Hydrochlorid der (*E*)-6-Amino-2-fluor-hex-2-ensäure,
- dem Hydrochlorid der 7-Amino-2-fluor-hept-2-ensäure in Form einer Mischung der Z- und E-Isomere,
- dem Hydrochlorid der (*Z*)-7-Amino-2-fluor-hept-2-ensäure,
- dem Hydrochlorid der 9-Amino-2-fluor-non-2-ensäure,
- dem Hydrochlorid der 10-Amino-2-fluor-dec-2-ensäure, und
- dem Hydrochlorid der 14-Amino-2-fluor-tetradec-2-ensäure.

**9.** Derivate von ungesättigten Aminofettsäuren gemäß der allgemeinen Formel (I), wie in einem der Ansprüche 1 bis 8 definiert, als neue chemische Verbindungen, mit Ausnahme der folgenden Verbindungen: 6-Amino-hex-2-ensäure, deren Hydrochlorid und Trifluoracetat, 11-Amino-2-undecensäure, das Trifluoracetat der 8-Amino-oct-2-ensäure, 8-(Dimethylamino)-oct-2-ensäure, 12-(Dimethlyamino)-dodec-2-ensäure, 6-(Isopropylamino)-2-methyl-hex-2-ensäure-Ethylester, 6-(*tert*-Butylamino)-2-methyl-hex-2-ensäure-Ethylester, 6-Amino-2-methyl-hex-2-ensäure-Methylester, 7-Amino-hept-2-ensäure-Methylester, 7-Amino-2-methyl-hept-2-ensäure-Methylester und 7-Amino-4-methyl-hept-2-ensäure-Methylester.

**10.** Dermatologische Zusammensetzungen, umfassend als aktives Agens mindestens ein Derivat einer ungesättigten Aminofettsäure nach einem der Ansprüche 1 bis 8, in Verbindung mit einem dermatologisch akzeptablen Träger.

**11.** Verwendung eines Derivats einer ungesättigten Aminofettsäure nach einem der Ansprüche 1 bis 8 zur Herstellung einer dermokosmetischen Zusammensetzung, die wirksam ist gegen Radikale, Entzündungen, Juckreiz, Kollagenase und/oder die bestimmt ist zur Behandlung der Hautalterung und/oder zur Behandlung von Problemen der Keratinisation und der Pigmentierung und/oder zur Verbesserung der Wundheilung.

**12.** Verwendung durch Anspruch 11, **dadurch gekennzeichnet, dass** die Zusammensetzung bestimmt ist zur Behandlung der Psoriasis, des Juckreizes und/oder der atopischen Dermatitis; oder zur Repigmentierung der Haare oder der Haut, insbesondere der weißen Altersflecken; oder zum Bewirken einer Aufhellung der Haut oder zur Behandlung der braunen Altersflecken.

**13.** Verfahren zur Herstellung eines Derivats einer ungesättigten Aminofettsäure der folgenden allgemeinen Formel (I):

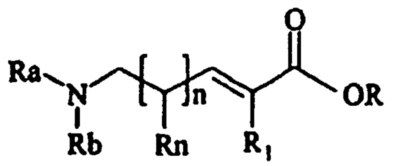

(I)

wobei :

- Rn jeweils unabhängig voneinander ein Wasserstoffatom oder eine lineare oder verzweigte Alkylgruppe mit 1 bis 6 Kohlenstoffatomen repräsentiert, ggf. substituiert durch ein oder mehrere Halogenatome, insbesondere Fluoratome;
- $R_1$ ein Wasserstoff-, Fluor-, Chlor- oder Bromatom repräsentiert, oder auch eine $CF_3$- oder $CHF_2$-Gruppe oder auch eine lineare oder verzweigte Alkylgruppe, Alkenylgruppe mit mindestens einer Doppelbindung oder Alkinylgruppe mit mindestens einer Dreifachbindung, umfassend 1 bis 10 Kohlenstoffatome, ggf. substituiert durch ein Halogenatom, insbesondere ein Fluoratom;
- R ein Wasserstoffatom oder eine Gruppe R' repräsentiert, welchletztere eine lineare oder verzweigte Alkylgruppe mit 1 bis 6 Kohlenstoffatomen oder eine Cycloalkylgruppe mit 1 bis 6 Kohlenstoffatomen repräsentiert, ggf. substituiert durch ein Halogenatom, insbesondere ein Fluoratom;
- Ra und Rb unabhängig voneinander ein Wasserstoffatom oder eine lineare oder verzweigte Alkyl- oder Acylgruppe mit 1 bis 6 Kohlenstoffatomen repräsentieren, wobei Ra und Rb gemeinsam einen ringförmigen Kohlenwasserstoff mit 4 bis 6 Kohlenstoffatomen bilden können; und
- n eine ganze Zahl zwischen 2 und 14 ist;

wobei das Herstellungsverfahren umfasst:

- die Durchführung der Wittig-Horner-Reaktion durch die Umsetzung eines Phosphonats der folgenden allgemeinen Formel (II):

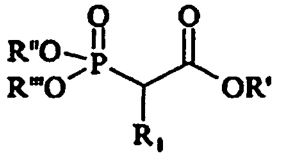

(II)

wobei:

- $R_1$ wie oben definiert ist,
- R' eine lineare oder verzweigte Alkylgruppe mit 1 bis 6 Kohlenstoffatomen repräsentiert, vorzugsweise eine Ethylgruppe, und
- R" und R"' unabhängig voneinander eine lineare oder verzweigte Alkylgruppe mit 1 bis 6 Kohlenstoffatomen repräsentieren, bevorzugt eine Ethyl- oder Methylgruppe, wobei die Gruppen R" und R"' einen ringförmigen Kohlenwasserstoff mit 2 bis 4 Kohlenstoffatomen bilden können,

oder, wenn $R_1$ = H, die Durchführung der Doebner-Knoevenagel-Reaktion durch die Umsetzung eines Malonsäurederivats der Formel $R'OOC\text{-}CH_2\text{-}COOR'$, wobei R' wie oben definiert ist,
mit einer Verbindung der folgenden allgemeinen Formel (III):

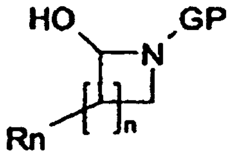

(III)

wobei:

• n wie oben definiert ist, und
• GP eine Schutzgruppe repräsentiert, wobei N-GP vorteilhafterweise eine Gruppe vom Typ Carbamat repräsentiert, bevorzugt ein tert-Butylcarbamat oder ein Benzylcarbamat,

um eine Verbindung der folgenden allgemeinen Formel (IV) zu erhalten:

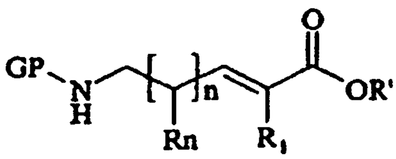

(IV)

bei der GP, R', $R_1$, Rn und n wie oben definiert sind,

- eine eventuelle Verseifungsreaktion der oben genannten Verbindung der allgemeinen Formel (IV), um eine Verbindung der folgenden allgemeinen Formel (V) zu erhalten:

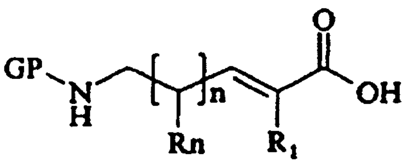

(V)

bei der GP, $R_1$, Rn und n wie oben definiert sind,

- eine Entschützungsreaktion des Stickstoffs der oben genannten Verbindung der Formel (IV) oder (V), um eine Verbindung der Formel (I) zu erhalten, bei der Ra und Rb ein Wasserstoffatom repräsentieren,
- und eine eventuelle N-Alkylierungs- oder N-Acylierungsreaktion der vorhergehenden Verbindung der Formel (I), bei der Ra und Rb ein Wasserstoffatom repräsentieren, um eine Verbindung der Formel (I) zu erhalten, bei der mindestens eine der Gruppen Ra und Rb kein Wasserstoffatom repräsentiert.

**14.** Verfahren nach Anspruch 13, **dadurch gekennzeichnet, dass** die Verbindung der allgemeinen Formel (III):

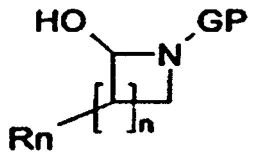

(III)

ausgehend von einer Verbindung der allgemeinen Formel (VI) synthetisiert wird:

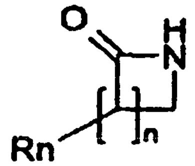

(VI)

bei der n und Rn wie in Anspruch 13 definiert sind,
gemäß den folgenden Schritten:

- Schützen des Stickstoffs der Amidfunktion der Verbindung der Formel (VI) durch eine Gruppe GP, um eine Verbindung der folgenden allgemeinen Formel (VII) zu erhalten:

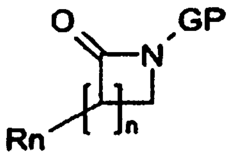

(VII)

bei der n, Rn und GP wie in Anspruch 13 definiert sind,

- nachfolgend Reduktion der Carbonylfunktion der Verbindung der Formel (VII) wie oben definiert, wobei dieser Schritt insbesondere mit Hilfe von Aluminiumhydriden, wie etwa dem Diisobutylaluminiumhydrid, bei tiefer Temperatur durchgeführt wird.

**15.** Verbindungen der allgemeinen Formel (VIII) als Synthesezwischenprodukte:

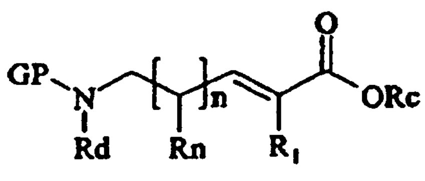

(VIII)

wobei:

• Rc ein Wasserstoffatom oder eine lineare oder verzweigte Alkylgruppe mit 1 bis 6 Kohlenstoffatomen repräsentiert;
• $R_1$ ein Wasserstoff-, Fluor-, Chlor- oder Bromatom repräsentiert, oder auch eine $CF_3$- oder $CHF_2$-Gruppe oder auch eine lineare oder verzweigte Alkylgruppe, Alkenylgruppe mit mindestens einer Doppelbindung oder Alkinylgruppe mit mindestens einer Dreifachbindung, umfassend 1 bis 6 Kohlenstoffatome, ggf. substituiert durch ein oder mehrere Halogenatome, insbesondere Fluoratome;

- Rn jeweils unabhängig voneinander ein Wasserstoffatom oder eine lineare oder verzweigte Alkylgruppe mit 1 bis 6 Kohlenstoffatomen repräsentiert, ggf. substituiert durch ein oder mehrere Wasserstoffatome, insbesondere Fluoratome;
- n eine ganze Zahl zwischen 2 und 14 ist;
- GP eine Schutzgruppe repräsentiert, wobei N-GP vorteilhafterweise eine Gruppe vom Typ Carbamat repräsentiert, bevorzugt ein *tert*-Butylcarbamat oder ein Benzylcarbamat; und
- Rd ein Wasserstoffatom repräsentiert,

mit Ausnahme der folgenden Verbindungen:
(*E*)-6-(*N,N*-Di-*tert*-butoxy-carbonylamino)-hex-2-ensäure-*tert*-Butylester, (*E*)-6-(*N*-*tert*-Butoxy-carbonylamino)-hex-2-ensäure-*tert*-Butylester, (*E*)-6-(*N*-*tert*-Butoxy-carbonylamino)-hex-2-ensäure-Ethylester, (*E*)-7-(*N*-*tert*-Butoxy-carbonylamino)-hept-2-ensäure-Methylester und (*E*)-7-(*N*-*tert*-Butoxy-carbonylamino)-hept-2-ensäure.

**16.** Verbindung nach Anspruch 15, **dadurch gekennzeichnet, dass** Rn ein Wasserstoffatom repräsentieren.

**17.** Verfahren nach Anspruch 16, **dadurch gekennzeichnet, dass** $R_1$ ein Wasserstoff- oder Fluoratom repräsentiert.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005068643 A
- WO 9104746 A
- FR 2829491


### Littérature non-brevet citée dans la description


- **DAVIES et al.** *Tetrahedron : Asymmetry,* 2006, vol. 17, 1793-1811
- **MENTINK et al.** *Org. Lett.,* 2002, vol. 4 (20), 3497-1500
- **DE STAMMER ; WEBB.** *J. Org. Chem.,* 1969, vol. 34 (8), 2306-2311
- **DE MARSZAK ; OLOMUCKI.** *Bull. Soc. Chim. Fr.,* 1959, 182-185
- **DE GUINDON et al.** *Org. Lett.,* 2001, vol. 3 (15), 2293-2296
- **KNOUZI et al.** *Tetrahedron Lett.,* 1987, vol. 28 (16), 1757-1760
- **DE PERRON et al.** *Tetrahedron Lett.,* 2003, vol. 44, 6553-6556
- **DE CACCIOLA et al.** *Tetrahedron Lett.,* 1997, vol. 38 (33), 5741-5744
- **THEODORA GREEN ; PETER WUTS.** Protective Groups in Organic Synthesis. Wiley Interscience, 17-246
- **DE LIST et al.** *Adv. Synth. Catal.,* 2005, vol. 347, 1558-1560
- **MICHAEL.B. SMITH ; JERRY MARCH.** March's Advanced Organic Chemistry. 501-552
- **MICHAEL.B. SMITH ; JERRY MARCH.** March's Advanced Organic Chemistry. vol. 1381
- **THEODORA GREEN ; PETER WUTS.** Protective Groups in Organic Synthesis. Wiley Interscience, 494-654
- **T.KUNIEDA ; T.HIGUCHI ; Y.ABE ; M. HIROBE.** *Chem. Pharm. Bull.,* 1984, vol. 32, 2174
- **I. GRAPSAS ; Y.J.CHO ; S.MOBASHERY.** *J. Org. Chem.,* 1994, vol. 59, 1918
- **LOUIS FIESER ; MARY FIESER.** *Reagents for organic synthesis,* vol. I, 3-6
- **THEODORA GREEN ; PETER WUTS.** Protective Groups in Organic Synthesis. Wiley Interscience, 17-246
- **SUEMATSU M et al.** Free Radicals Pratical Approach. 1996, 83-99
- **JANSSEN Y, M, W et al.** *Lab, Invest,* 1993, vol. 69, 261-74
- **VAN DER VLIET A ; BAST A.** *Chem, Biol, Interactions,* 1992, vol. 85, 95-116
- **YU B, P.** *Physiol, Rev,* 1994, vol. 74, 139-62
- **STEILING H et al.** *Exp. Cell, Res,* 1999, vol. 247, 484-94
- **MIYACHI Y et al.** *J, Clin, Lab, Immunol,* 1986, vol. 19, 11-4
- **KRESS M et al.** *Pain,* 1995, vol. 62, 87-94
- **GIROTTI A, W.** *J, Free Radic, Biol, Med,* vol. 1, 87-95
- **VESSEY D, A et al.** *J, Invest, Dermatol,* 1992, vol. 99, 859-63
- **MORLIÈRE P et al.** *Biochim, Biophys, Acta,* 1991, vol. 1084, 261-268
- **EMONET E et al.** *J. Photochem. Photobiol,* 1997, vol. 40, 84-90
- **MEUN, Y. J. et al.** *Biol. Pharm. Bull.,* Juin 2004, vol. 27 (6), 806-809
- **CURTO EV. ; KWONG C. ; HERMERSDÖRFER H. ; GLATT H. ; SANTIS C. ; VIRADOR V. ; HEARING VJ. ; DOOLEY TP.** Inhibitors of mammalian melanocyte tyrosinase: in vitro comparisons of alkyl esters of gentisic acid with others putative inhibitors. *Biochemical Pharmacology,* 1999, vol. 57, 663-672
- **JIMBOW K. ; HUA C. ; GOMEZ P. ; HIROSAKI K. ; SHINODA K. ; SALOPEK T. ; MATSUSAKA H. ; JIN H. ; YAMASHITA T.** Intracellular vesicular trafficking of tyrosinase gene family protein in eu- and pheomelanosome biogenesis. *Pigment cell res,* 2000, vol. 13, 110-117